# EUROPEAN PATENT SPECIFICATION

(11) **EP 3 333 011 B1**
(45) Date of publication and mention of the grant of the patent: **10.03.2021**
(21) Application number: 17206306.7
(22) Date of filing: 09.12.2017
(51) Int. Cl.: B60N 2/56, B60N 2/66, B60W 50/00, B60N 2/00, B60N 2/90, B60N 2/02

(54) **MOTION SICKNESS MITIGATION**
REISEKRANKHEITSABSCHWÄCHUNG
ATTÉNUATION DU MAL DES TRANSPORTS

(30) Priority: 09.12.2016 US 201662432245 P
(43) Date of publication of application: 13.06.2018
(73) Proprietor: Faurecia Automotive Seating, LLC, Auburn Hills, MI 48326 (US)
(72) Inventor: Ketels, Cedric, Holland, Michigan 49424 (US); Goodrich, Rod, Watervliet, Michigan 49098 (US); Benson, Matthew K., Holland, Michigan 49424 (US); Bransdorfer, Alfred H., Holland, Michigan 49424 (US)
(74) Representative: Kiriczi, Sven Bernhard

(56) References cited:
- DE-A1-102015 117 980
- US-A1- 2015 105 641

## Description

### PRIORITY CLAIM

This application claims priority to U.S. Provisional Application Serial No. 62/432,245, filed December 9, 2016.

### BACKGROUND

The present disclosure relates to vehicle systems. More particularly, the present disclosure relates to vehicle systems for use in providing a comfortable environment for an occupant of the vehicle.

### SUMMARY

According to the present disclosure, a vehicle is adapted for movement along a roadway. A cabin of the vehicle is adapted for surrounding and supporting an occupant during movement of the vehicle. Systems of the vehicle control an environment within the cabin.

In illustrative embodiments, a motion-sickness mitigation system includes a control system and a sensory feedback system. The control system monitors for motion inputs to identify changes in movement of the vehicle. The control system is configured to produce a signal indicative of the change in movement. The sensory feedback system generates at least one of a tactile, visual, audial, and olfactory alert indicative of the change in movement to inform an occupant of the vehicle of the change in movement in response to receiving the signal from the control system to reduce a likelihood of the occupant becoming motion-sick.

According to another aspect of the present disclosure, a vehicle includes an occupant support mounted to a floor of the vehicle. The occupant support includes a seat base coupled with the vehicle for movement with the vehicle and a seat coupled to the seat base. The seat is adapted to support an occupant of the occupant support on a support surface above the floor of the vehicle.

In illustrative embodiments not forming part of the invention, the occupant support further includes means for facilitating relative movement between the support surface of the seat and the seat base in response to an acceleration of the seat base to reduce acceleration of the occupant during acceleration of the seat base so that motion sickness experienced by the occupant is minimized. The means is configured to reduce the magnitude of at least one of a lateral acceleration and a fore-and-aft acceleration of the occupant during the acceleration of the seat base.

In illustrative embodiments not forming part of the invention , the means includes a plurality of actuators and a controller. The actuators are coupled with the seat and the seat base. The controller is configured to control movement of each of the plurality of actuators individually to cause the plurality of actuators to move the seat relative to the seat base in response to the acceleration of the seat base. In illustrative embodiments not forming part of the invention, the controller is configured to translate and tilt the seat relative to the seat base in response to the seat base accelerating.

In illustrative embodiments not forming part of the invention, the seat includes a left lateral side, a right lateral side, a fore end, and an aft end. The controller is configured to cause the plurality of actuators to raise the left lateral side and lower the right lateral side of the seat in response to the vehicle experiencing a right turn. The controller is configured to cause the plurality of actuators to translate the seat in the aft direction in response to the vehicle accelerating in the forward direction.

Additional features of the present disclosure will become apparent to those skilled in the art upon consideration of illustrative embodiments exemplifying the best mode of carrying out the disclosure as presently perceived.

### BRIEF DESCRIPTIONS OF THE DRAWINGS

The detailed description particularly refers to the accompanying figures in which:

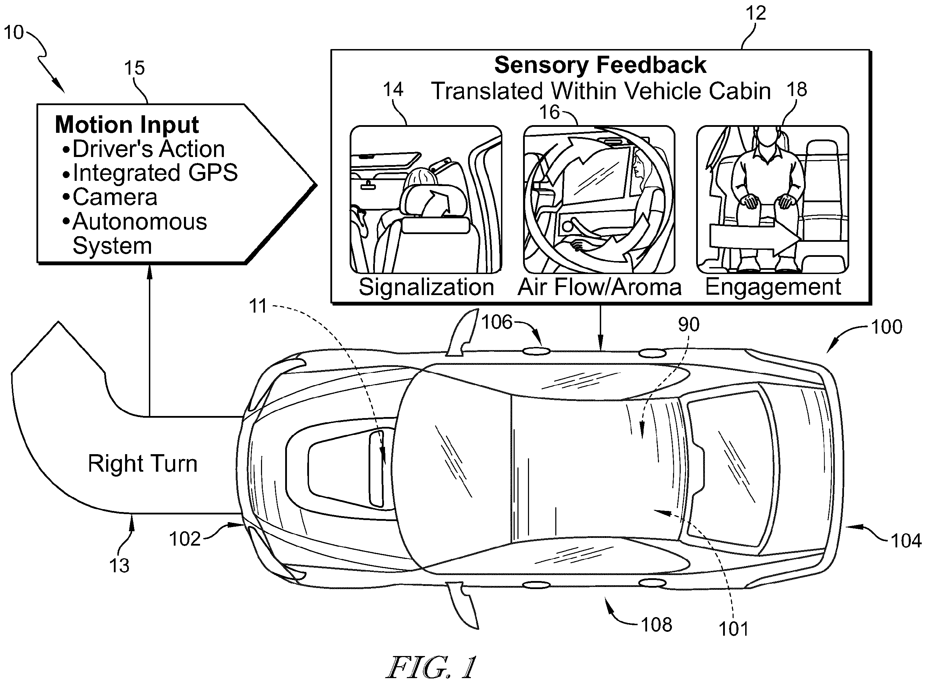

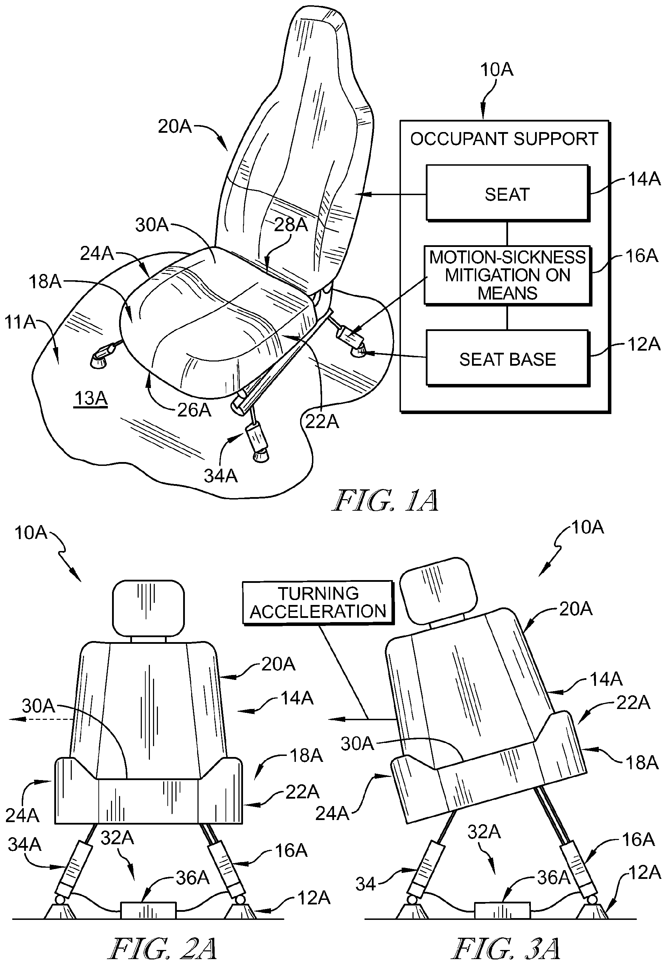

Fig. 1 is a diagrammatic view of a motion-sickness mitigation system used to increase comfort of an occupant in a cabin of a vehicle by providing sensory feedback to the occupant in response to motion input of the vehicle to alert the occupant to changes in motion of the vehicle to reduce a likelihood that the occupant becomes motion-sick due to changes in movement of the vehicle;

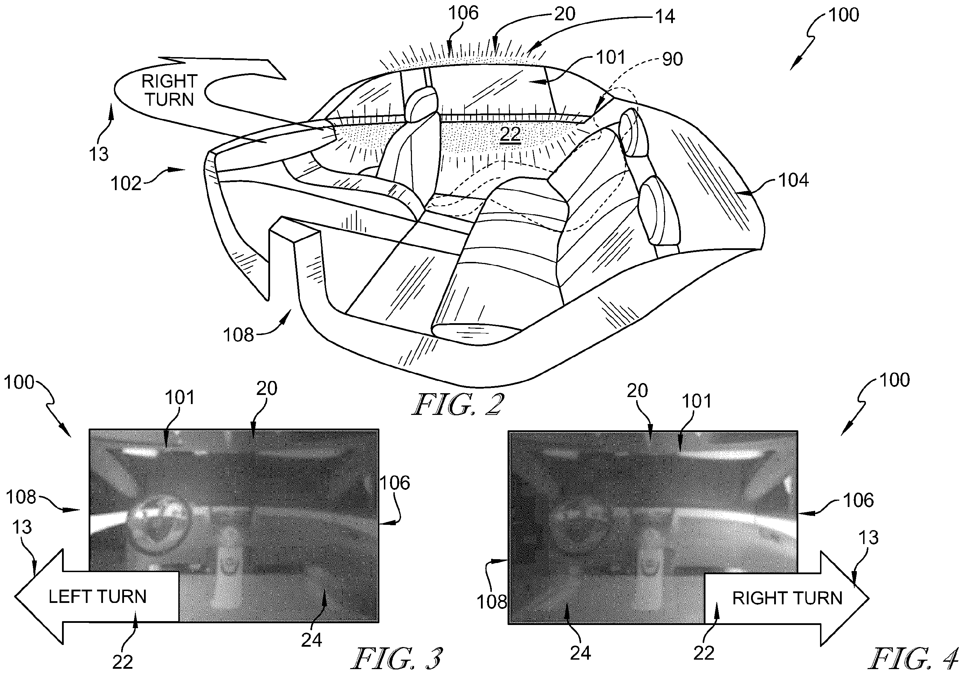

Fig. 2 is a diagrammatic and perspective view of one embodiment of a process for providing sensory feedback in the vehicle of Fig. 1 showing a right-side portion of the cabin illuminated and suggesting that the right-side portion of the cabin becomes illuminated in response to an input of the vehicle signaling a right turn in order to indicate the change in movement to the occupant;
Fig. 3 is a diagrammatic and perspective view of another embodiment of a process for providing sensory feedback in the vehicle of Fig. 1 showing the right-side portion of the cabin dimmed compared to a left-side portion of the cabin and suggesting that the right-side portion of the cabin becomes dimmed in response to an input of the vehicle signaling a left turn in order to indicate the change in movement to the occupant;

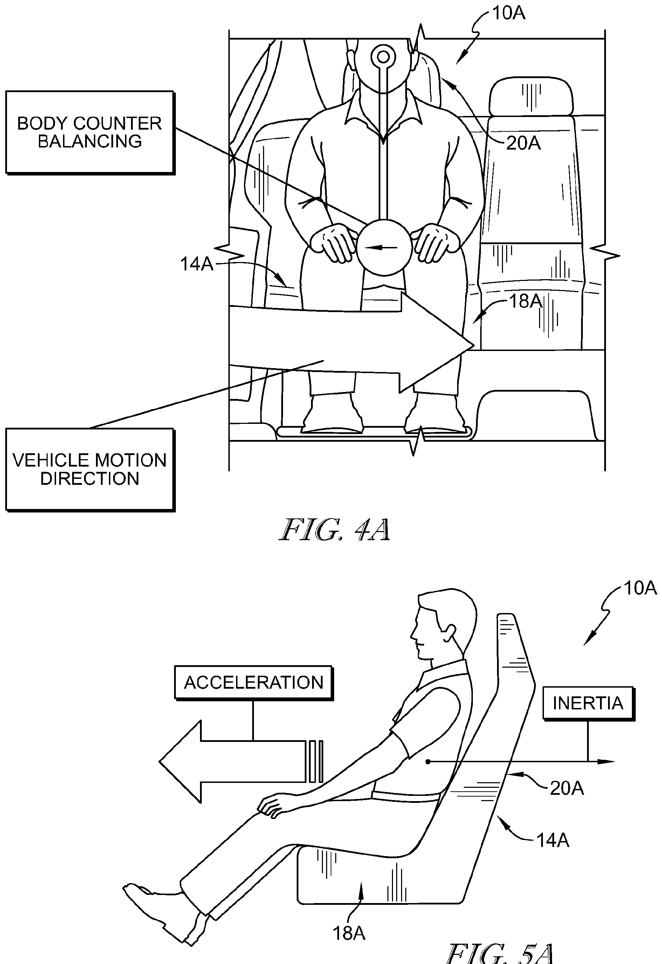

Fig. 4 is view similar to Fig. 3 showing the left-side portion of the cabin dimmed compared to the right-side portion of the cabin and suggesting that the left-side portion of the cabin becomes dimmed in response to an input of the vehicle signaling a right turn in order to indicate the change in movement to the occupant;

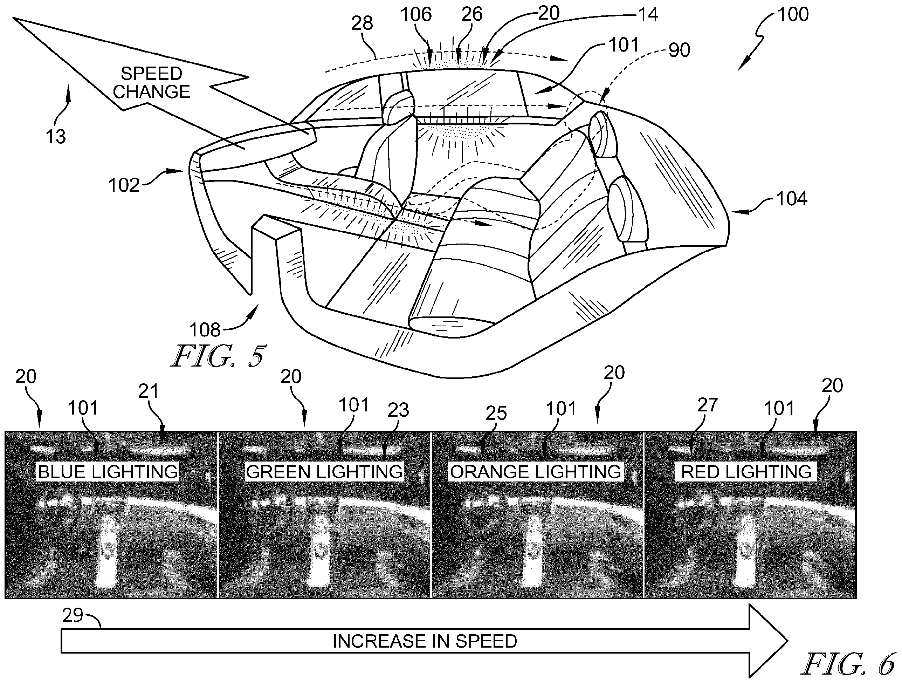

Fig. 5 is a diagrammatic and perspective view of another embodiment of a process for providing sensory feedback in the vehicle of Fig. 1 showing portions of the cabin illuminated and suggesting that illumination moves from a front of the cabin toward a rear of the cabin in response to an input of the vehicle signaling an increase in speed in order to indicate the change in movement to the occupant;

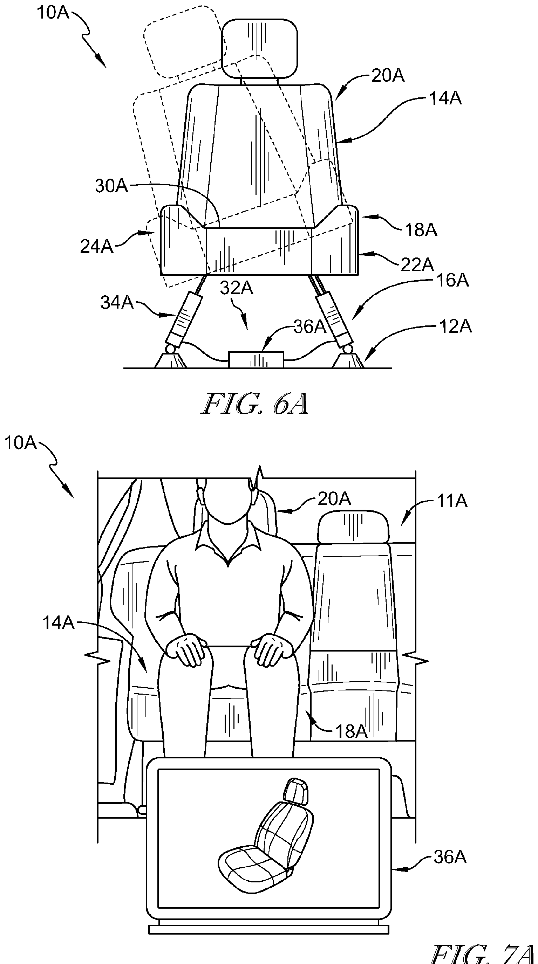

Fig. 6 is a diagrammatic and perspective view of another embodiment of a process for providing sensory feedback in the vehicle of Fig. 1 showing the cabin illuminated in a first color and suggesting that the illumination changes colors in response to an input of the vehicle signaling an increase in speed in order to indicate the change in movement to the occupant;

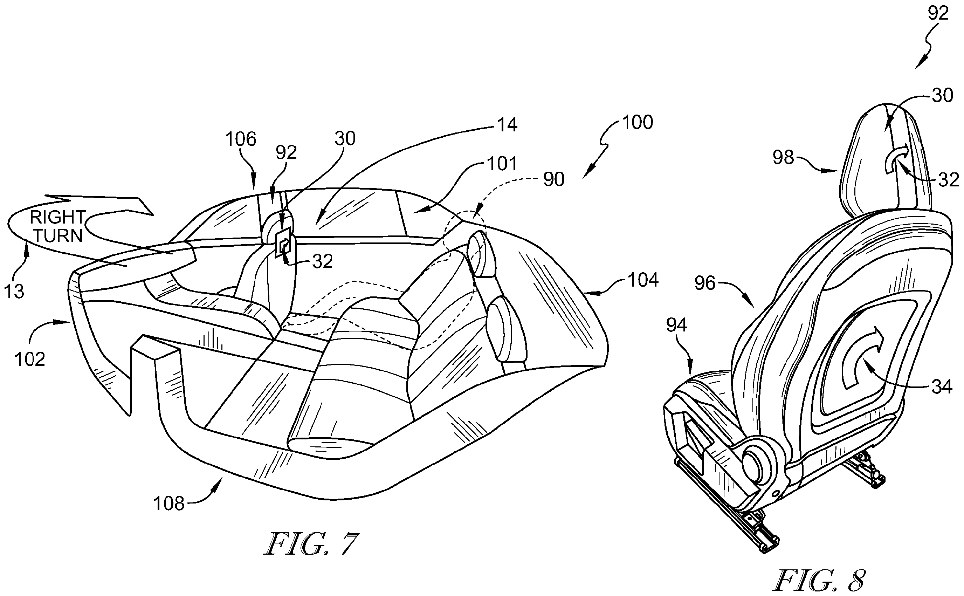

Fig. 7 is a diagrammatic and perspective view of another embodiment of a process for providing sensory feedback in the vehicle of Fig. 1 showing a right-turn arrow displayed on a monitor attached to a forward vehicle seat in the cabin and suggesting that the right-turn arrow is displayed on the monitor in response to an input of the vehicle signaling a right turn in order to indicate the change in movement to the occupant;

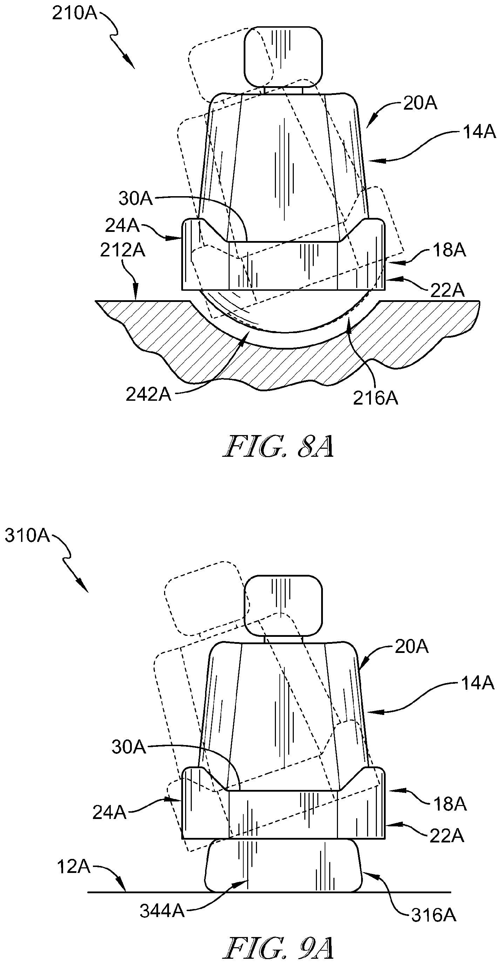

Fig. 8 is a perspective view of a vehicle seat of the vehicle in Fig. 1 showing illuminated arrows on a seat back and head rest of the vehicle seat and suggesting that that the arrows become illuminated in response to an input of the vehicle signaling a turn in order to indicate the change in movement to the occupant;

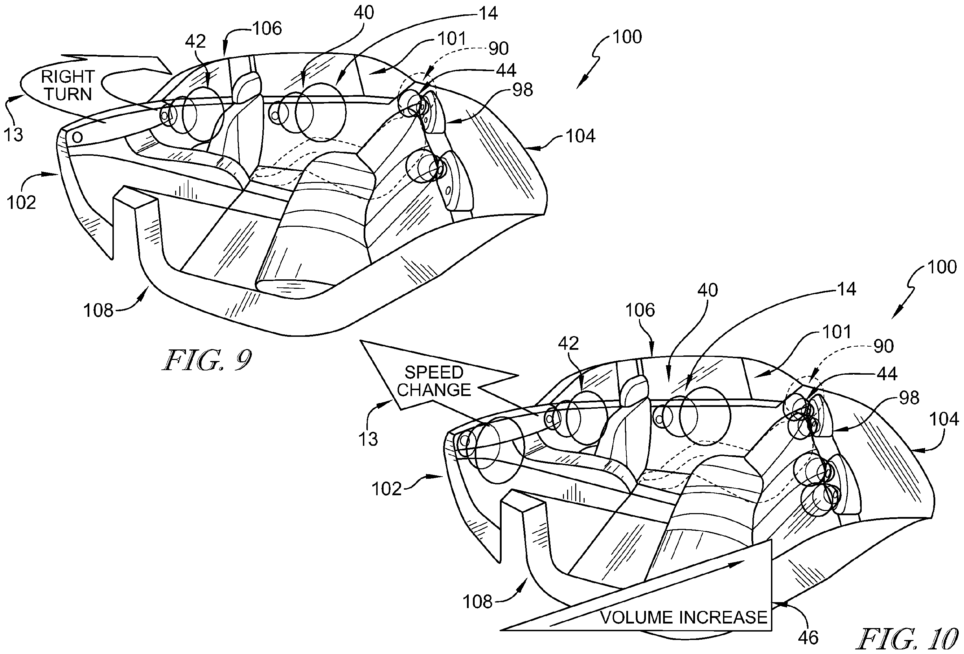

Fig. 9 is a diagrammatic and perspective view of another embodiment of a process for providing sensory feedback in the vehicle of Fig. 1 showing a sound system of the cabin and suggesting that the sound system produces sound along the right-side portion of the cabin and right-side portions of the head rests in response to an input of the vehicle signaling a right turn in order to indicate the change in movement to the occupant;

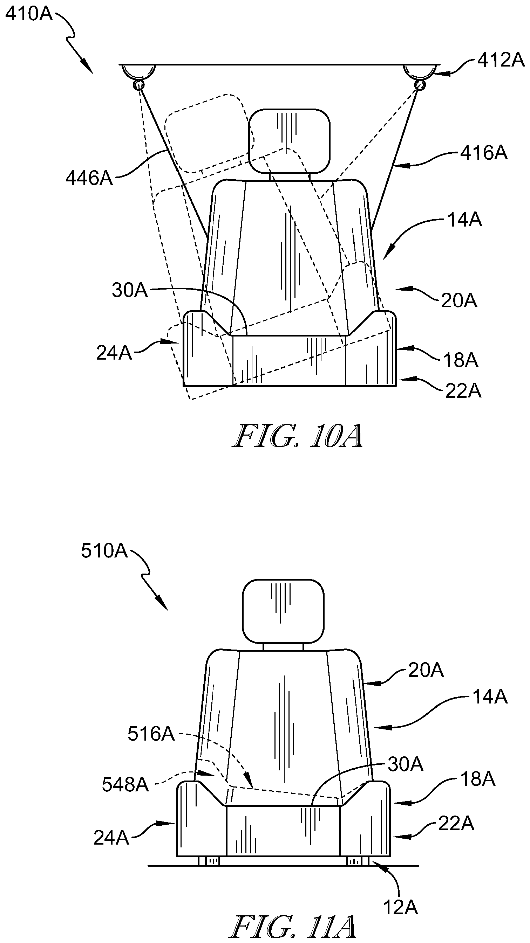

Fig. 10 is a diagrammatic and perspective view of another embodiment of a process for providing sensory feedback in the vehicle of Fig. 1 showing the sound system of the cabin and suggesting that the sound system produces sound of increasing volume in response to an input of the vehicle signaling an increase in speed in order to indicate the change in movement to the occupant;

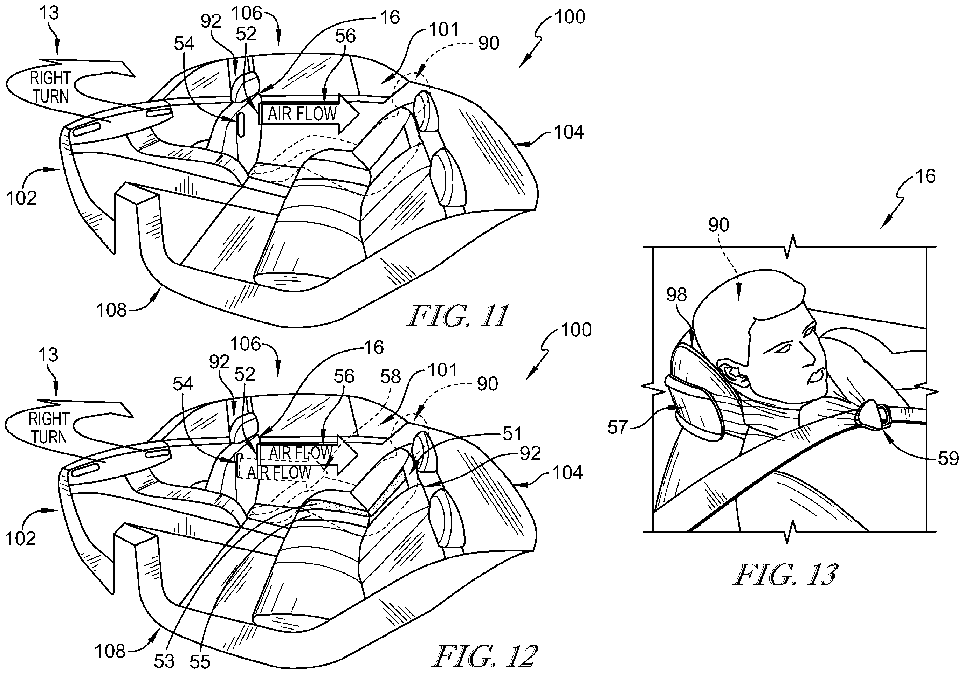

Fig. 11 is a diagrammatic and perspective view of another embodiment of a process for providing sensory feedback in the vehicle of Fig. 1 showing that fans are included in the forward vehicle seat and suggesting that air flow is produced by a right-side fan toward the occupant in response to an input of the vehicle signaling a right turn in order to indicate the change in movement to the occupant;
Fig. 12 is a view similar to Fig. 11 showing that a variable-temperature mat is included in an occupant support surface of the rear vehicle and suggesting that relatively warmer air flow is produced by a right-side fan than a left-side fan toward the occupant and a right-side of the mat is relatively warmer than a left-side in response to an input of the vehicle signaling a right turn in order to indicate the change in movement to the occupant;
Fig. 13 is a perspective view of the occupant in a vehicle seat of the vehicle of Fig. 1 showing that one or more fan ducts are included in a headrest of the vehicle seat and that a fan is included on a restraint of the vehicle and suggesting that the air flow is produced by the fan ducts and fan of the restraint to indicate changes in vehicle movement to the occupant;

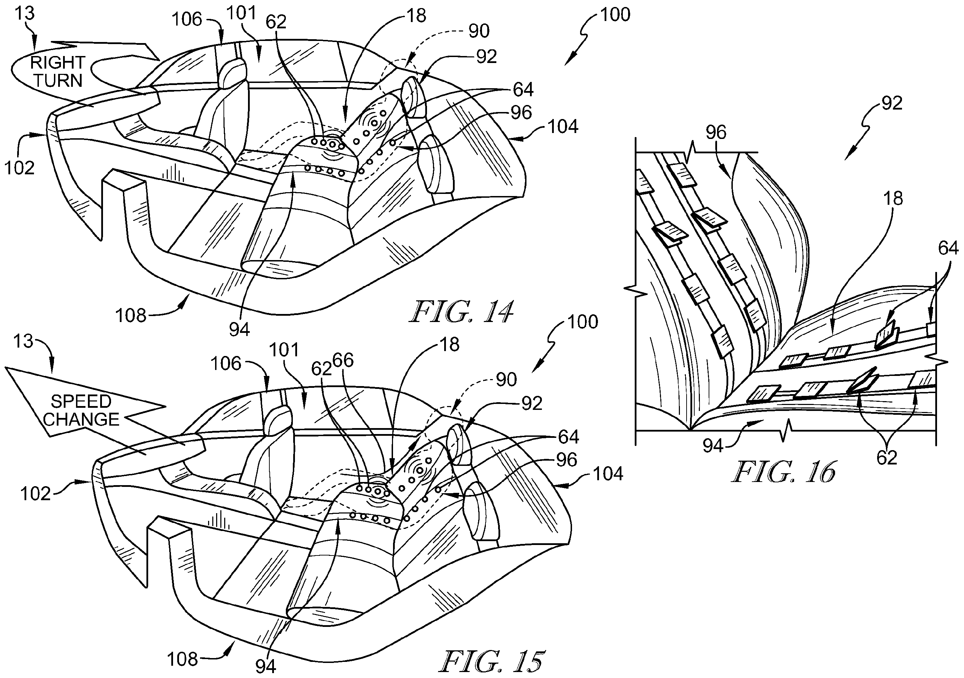

Fig. 14 is a diagrammatic and perspective view of another embodiment of a process for providing sensory feedback in the vehicle of Fig. 1 showing motion points included in the support surface the rear vehicle seat and suggesting that motion points along a right-side portion of the vehicle seat engage with the occupant in response to an input of the vehicle signaling a right turn in order to indicate the change in movement to the occupant;
Fig. 15 is a view similar to Fig. 14 showing the motion points of the vehicle seat and suggesting that the motion points engage with the occupant from front to back in response to an input of the vehicle signaling an increase in speed in order to indicate the change in movement to the occupant;
Fig. 16 is a perspective view of one embodiment of a vehicle seat showing that the vehicle seat includes a seat bottom and a seat back and that motion points in the form of inflatable bladders are included along the seat bottom and seat back;

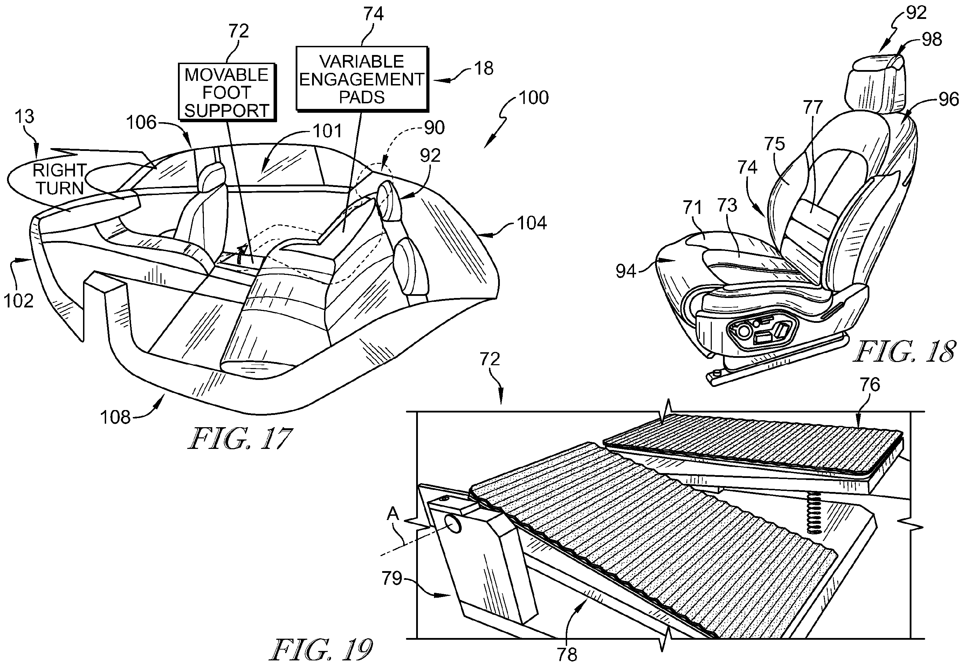

Fig. 17 is a diagrammatic and perspective view of another embodiment of a process for providing sensory feedback in the vehicle of Fig. 1 showing that variable engagement pads are included in the occupant support surface of the rear vehicle and that a movable foot support is positioned on a floor of the vehicle and suggesting that variable engagement pads along a right side of the vehicle seat increase engagement with the occupant and the movable foot support tilts toward a right side of the vehicle in response to an input of the vehicle signaling a right turn in order to indicate the change in movement to the occupant;
Fig. 18 is a perspective view of one embodiment of a vehicle seat showing that the vehicle seat includes a seat bottom and a seat back and that variable engagement pads are included in right-side and left-side bolsters of the seat bottom and seat back and in central portions of the seat bottom and seat back;
Fig. 19 is a perspective view of one embodiment of a movable foot support showing that the movable foot support includes two independently adjustable pedals that move relative to a base;

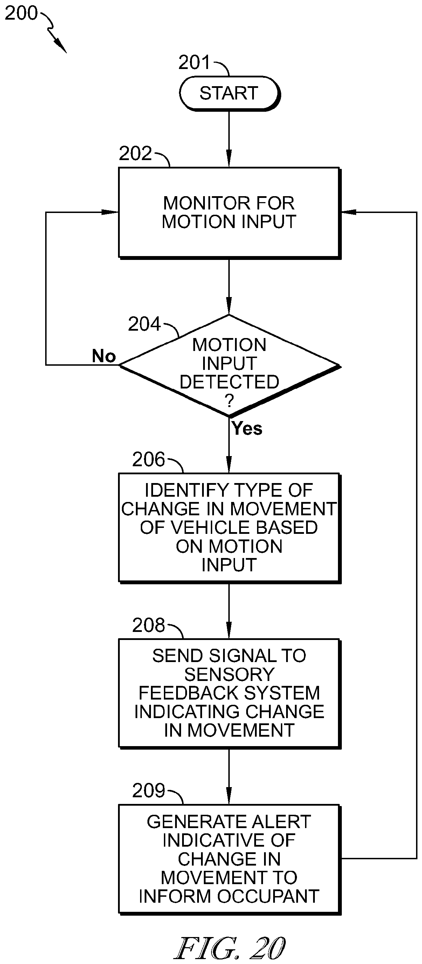

Fig. 20 is a diagrammatic view of one embodiment of a process for providing sensory feedback in the vehicle of Fig. 1;
Fig. 1A is a perspective and diagrammatic view of an occupant support adapted for use in a passenger vehicle showing that the occupant support includes a seat base, a seat, and motion-sickness mitigation means coupled with the seat base and the seat, the motion-sickness mitigation means includes a plurality of actuators configured to move the seat relative to the seat base in response to an acceleration of the seat base to reduce, remedy, or prevent motion sickness of the occupant;
Fig. 2A is a diagrammatic view of the occupant support of Fig. 1 showing that the seat is upright and that the seat base is not experiencing an acceleration;
Fig. 3A is a diagrammatic view of the occupant support of Fig. 1 suggesting that the occupant support is experiencing a right-turn acceleration and showing that the motion-sickness mitigation means has moved the seat relative to the seat base so that the seat is tilted in response to the acceleration being applied to the occupant support;
Fig. 4A is a diagrammatic view of an occupant supported by the occupant support suggesting that the occupant's body experiences inertia against an acceleration caused by the vehicle making a turn;
Fig. 5A is a diagrammatic view of an occupant supported by the occupant support suggesting that the occupant's body experiences inertia force against a forward acceleration of the occupant support;
Fig. 6A is a diagrammatic view of the occupant support of Fig. 1 showing that the motion-sickness mitigation means includes a plurality of actuators configured to tilt and translate the seat relative to the seat base in response to an acceleration of the seat base;
Fig. 7A is a diagrammatic view of the occupant support showing that the occupant support includes a controller configured to make adjustments manually by the occupant or automatically by a vehicle system by using the controller;
Fig. 8A is a diagrammatic view of another not forming part of the invention of an occupant support showing that the occupant support includes a seat, motion-sickness mitigation means, and a seat base wherein the motion-sickness mitigation means includes a rotatable seat mount configured to rotate the seat relative to the seat base in response to an acceleration of the seat base to minimize motion sickness of an occupant of the occupant support;
Fig. 9A is a diagrammatic view of another embodiment not forming part of the invention of an occupant support showing that the occupant support includes a seat, motion-sickness mitigation means, and a seat base wherein the motion-sickness mitigation means includes a bias member coupled with the seat and the seat base and configured to allow the seat to move relative to the seat base in response to an acceleration of the seat base to minimize motion sickness of an occupant of the occupant support;
Fig. 10A is a diagrammatic view of another embodiment not forming part of the invention of an occupant support showing that the occupant support includes a seat, motion-sickness mitigation means, and a seat base wherein the motion-sickness mitigation means includes a strap coupled with the seat and the seat base to suspend the seat from the seat base to allow the seat to move relative to the seat base in response to an acceleration of the seat base to minimize motion sickness of an occupant of the occupant support; and
Fig. 11A is a diagrammatic view of another embodiment not forming part of the invention of an occupant support showing that the occupant support includes a seat, motion-sickness mitigation means, and a seat base wherein the motion-sickness mitigation means includes an adjustable seat cushion configured to move the seat relative to the seat base in response to an acceleration of the seat base to minimize motion sickness of an occupant of the occupant support.

### DETAILED DESCRIPTION

A motion-sickness mitigation system 10 in accordance with the present invention is adapted for use in a vehicle 100 as shown in Fig. 1. Motion-sickness mitigation system 10 is configured to increase comfort of an occupant 90 in a cabin 101 of vehicle 100 by providing sensory feedback to occupant 90 in response to motion inputs 15 of vehicle 100 to alert occupant 90 to changes in movement 13 of vehicle 100, such as a right turn, to reduce a likelihood that occupant 90 becomes motion-sick due to changes in movement 13 of vehicle 100. For example, a sensory feedback system 12 includes at least one of a signalization system 14 (Figs. 2-10), an air flow system 16 (Figs. 11-13), and an engagement system 18 (Figs. 14-19) to generate one or more of a tactile, visual, audial, and olfactory alert indicative of change in movement 13 of vehicle 100 to inform occupant 90. One embodiment of a process 200 for generating the alerts is shown in Fig. 20.

Motion-sickness mitigation system 10 includes a control system 11 and sensory feedback system 12 as shown in Fig. 1. Control system 11 is configured to identify changes in movement 13 (such as a right turn) of vehicle 100 based on motion inputs 15 detected by control system 11. In some embodiments, control system 11 is integrated with other systems used to control operation of vehicle 100.

In the illustrative embodiment, motion inputs 15 include at least one of a concurrent, an anticipated, or an instructed change in movement 13 of vehicle 100 as suggested in Fig. 1. For example, a driver input, such as turning a steering wheel or depressing a brake pedal of vehicle 100, generates a concurrent change in movement 13 of vehicle 100. Integrated systems of vehicle 100, such as a GPS or camera, can anticipate changes in movement 13. Other integrated systems of vehicle 100, such as an autonomous driving system, can instruct changes in movement 13 of vehicle 100 during an autonomous driving mode of vehicle 100.

Control system 11 identifies a type of change in movement 13 of vehicle 100, such as a right turn, a left turn, acceleration, or deceleration, and sends a signal to sensory feedback system 12 indicating change in movement 13 as suggested in Fig. 1. Sensory feedback system 12 in accordance with the present invention includes at least one of a signalization system 14, an air flow system 16, and an engagement system 18 to generate one or more alerts within cabin 101 indicative of change in movement 13 to inform occupant 90.

Signalization system 14 in accordance with the present invention can include one or more of a lighting system 20, indicator system 30, and sound system 40 to produce various types of alerts to inform occupant 90 of changes in motion 13 as suggested in Figs. 2-10. In one illustrative embodiment, lighting system 20 is configured to produce an illuminated portion 22 of cabin 101 to inform occupant 90 of change in movement 13 as shown in Fig. 2. For example, illuminated portion 22 extends along a right side 106 of cabin 101 indicative of a right turn of vehicle 100 as shown in Fig. 2. Similarly, lighting system 20 is configured to produce an illuminated portion 22 along a left side 108 of cabin 101 indicative of a left turn of vehicle 100. In some embodiments, lighting system 20 is also configured to produce a dimmed portion 24 along an opposite side of cabin 101 from illuminated portion 22 as suggested in Figs. 3 and 4. In some embodiments, illuminated portion 22 and/or dimmed portion 24 extend from a front 102 of cabin 101 to a rear 104 of cabin 101 to inform occupants 90 sitting in front-row seats and in back-row seats of vehicle 100 of change in movement 13.

In another illustrative embodiment, lighting system 20 is configured to produce one or more moving points of illumination 26 to inform occupant 90 of change in movement 13 as suggested in Fig. 5. For example, points of illumination 26 move along cabin 101 from front 102 to rear 104 in a direction 28 to inform occupant 90 of acceleration of vehicle 100. Similarly, points of illumination can move from rear 104 to front 102 to inform occupant 90 of deceleration of vehicle 100. In yet another illustrative embodiment, lighting system 20 can produce an illuminated portion 22 along front 102 (indicative of acceleration) or rear 104 (indicative of deceleration) of cabin 101.

In yet another illustrative embodiment, lighting system 20 is configured to produce illumination of various colors to inform occupant 90 of changes in speed as suggested in Fig. 6. For example, lighting system 20 can illuminate cabin 101 with blue lighting 21 at low speeds, and illuminate cabin 101 with "warmer" colors (i.e., having a relatively longer wavelength in the electromagnetic spectrum) such as green 23, orange 25, and red 27, as the speed of vehicle 100 increases.

In some embodiments, cabin 101 is illuminated with first color indicating a steady-state in speed of vehicle 100, and illuminated with increasingly "cooler" colors (i.e., having relatively shorter wavelengths in the electromagnetic spectrum) to indicate deceleration or increasingly "warmer" colors to indicate acceleration of vehicle 100. In some embodiments, lighting system 20 is configured to produce various types of illumination within cabin 101, such as in a gradient pattern, gradually intensifying or subsiding brightness, pulsing or blinking, and color variations, among others.

In one illustrative embodiment, indicator system 30 is configured to produce indicia 32 visible to occupant 90 that is indicative of change in movement 13 as shown in Fig. 7. For example, indicator system 30 is configured to display a right curving arrow to inform occupant 90 of a right turn of vehicle 100. Similarly, indicator system 30 is configured to display a left curving arrow, an upward extending arrow, or a downward extending arrow to inform occupant 90 of a left turn, acceleration, or deceleration of vehicle 100, respectively. In some embodiments, indicator system is in the form of a monitor coupled to a front-row seat, such as vehicle seat 92 in Fig. 7. In some embodiments, a monitor is arranged in front of each seating position within cabin 101 as part of indicator system 30. While the indicia is shown in the form of stylized arrows, other forms of indicia are contemplated, such as a compass-style dial, moving graphics, patterns, and other forms of indicia useful for informing occupant 90 of changes in motion 13.

In another illustrative embodiment, indicator system 30 is integrated into vehicle seat 92 as suggested in Fig. 8. Vehicle seat 92 includes a seat bottom 94, a seat back 96 extending upward from seat bottom 94, and a head rest 98 attached to seat back 96. Indicator system 30 can produce one or more indicia 32, 34 in head rest 98 and seat back 96. In some embodiments, indicia 32, 34 are produced by an array of lights positioned inside vehicle seat 92 that illuminate in a pattern to form indicia 32, 34.

In one illustrative embodiment, sound system 40 is configured to produce sound waves 42 within cabin 101 that are indicative of change in movement 13 as shown in Fig. 9. For example, sound system 40 is configured to produce sound waves 42 along right side 106 of cabin 101 to inform occupant 90 of a right turn of vehicle 100. Similarly, sound system 40 is configured to produce sound waves 42 along left side 108 of cabin 101 indicative of a left turn of vehicle 100. In some embodiments, sound system 40 is integrated into an audio entertainment system of vehicle 100. In some embodiments, sound waves 42 are audible to occupant 90. In some embodiments, sound waves 42 create tactile pressure or vibration against occupant 90. In some embodiments, speakers are integrated into head rests 98, and sound waves 44 are produced thereby to alert occupant 90 to changes in motion 13.

In another illustrative embodiment, sound system 40 is configured to produce sound waves 42, 44 of increasing volume to indicate acceleration of vehicle 100 as shown in Fig. 10. Similarly, sound system 40 is configured to produce sound waves 42, 44 of decreasing volume to indicate deceleration of vehicle 100. In some embodiments, sound system 40 is configured to produce various types of audible and/or tactile sound waves 42, 44 in cabin 101, such as in a gradient pattern, gradually intensifying or subsiding volume, and pulsing, among others. In some embodiments, sound system 40 is configured to produce audible sound waves that mimic sounds produced by vehicles during various changes in motion, such as the screech of tires when turning.

One embodiment of air flow system 16 in accordance with the present disclosure is shown in Fig. 11. Air flow system 16 is configured to produce air flow toward occupant 90 indicative of change in movement 13. Air flow system 16 includes a right-side fan 52 and a left-side fan 54. In one illustrative embodiment, right-side fan 52 is configured to produce an air flow 56 along a right side of occupant 90 to inform occupant 90 of a right turn of vehicle 100. Similarly, left-side fan 54 is configured to produce an air flow along a left side of occupant 90 to inform occupant 90 of a left turn of vehicle 100. In some embodiments, air flow system 16 is configured to produce air flows of increasing or decreasing volume toward occupant 90 to indicate acceleration or deceleration of vehicle 100, respectively. In some embodiments, air flow system 16 is configured to produce a variety of aromas, such as lavender or ginger, to reduce a likelihood of motion sickness in occupant 90.

In another illustrative embodiment, air flow system 16 is configured to produce air flows having different temperatures to indicate change in movement 13 to occupant 90 as suggested in Fig. 12. For example, right-side fan 52 is configured to produce an air flow 56 along a right side of occupant 90 having a first temperature and left-side fan 54 is configured to produce an air flow 58 along a left side of occupant 90 having a second temperature lower than the first to inform occupant 90 of a right turn of vehicle 100. In some embodiments, the first and second temperatures are reversed, and this may depend on the temperature of the surrounding environment. In some embodiments, a variable-temperature mat 51 is integrated into vehicle seat 92. Variable-temperature mat 51 is configured to produce varying temperatures along a right-side portion 53 and a left-side portion 55 corresponding to the relative temperatures of air flows 56, 58.

As shown in Figs. 11 and 12, right-side and left-side fans 52, 54 are integrated into a front-row seat 92 of vehicle 100. In some embodiments, right-side and left-side fans 52, 54 are part of a HVAC system of vehicle 100 and integrated into other portions of cabin 101. In some embodiments, right-side and left-side fans 52, 54 are arranged in front of each seating position within cabin 101 as part of air flow system 16. In some embodiments, air flow system 16 includes fans 57 integrated into head rests 98 and a restraint fan 59 coupled to a restraint (e.g., seat belt) of vehicle 100 as shown in Fig. 13. Fans 57 can be operated in similar fashion to fans 52, 54. Restraint fan 59 is configured to direct air flow and/or aromas toward the face of occupant 90.

One embodiment of engagement system 18 in accordance with the present disclosure is shown in Fig. 14. Engagement system 18 is configured to produce tactile engagement with occupant 90 indicative of change in movement 13. Engagement system 18 includes a plurality of right-side motion points 62 arranged along a right side of vehicle seat 92 and a plurality of left-side motion points 64 arranged along a left side of vehicle seat 92. In one illustrative embodiment, right-side motion points 62 are configured to move and engage with occupant 90 to inform occupant 90 of a right turn of vehicle 100. Similarly, left-side motion points 64 are configured to move and engage with occupant 90 to inform occupant 90 of a left turn of vehicle 100.

In another illustrative embodiment, motion points 62, 64 engage with occupant 90 in sequence in a direction 66 from front 102 toward rear 104 of cabin 101 to indicate acceleration of vehicle 100 as suggested in Fig. 15. Similarly, motion points 62, 64 engage with occupant 90 in sequence in a direction from rear 104 toward front 102 of cabin 101 to indicate deceleration of vehicle 100. In some embodiments, motion points 62, 64 are in the form of inflatable bladders that expand and contract as shown in Fig. 16. In some embodiments, motion points 62, 64 are in the form of vibration mechanisms configured to generate vibrations.

Another embodiment of engagement system 18 in accordance with the present invention is shown in Fig. 17. Engagement system 18 is configured to produce tactile engagement with occupant 90 indicative of change in movement 13. Engagement system 18 includes at least one of a movable foot support 72 mounted to a floor of vehicle 100 and variable engagement pads 74 integrated into vehicle seat 92. Variable engagement pads 74 include seat-bottom bolster pads 71 and seat-bottom center pads 73 mounted to seat bottom 94, and seat-back bolster pads 75 and seat-back center pads 77 mounted to seat back 96 as shown in Fig. 18. Seat-bottom bolster pads 71 and seat-back bolster pads 75 are positioned along both sides of vehicle seat 92. Movable foot support 72 includes a right-side pedal 76 and a left-side pedal 78 mounted to a base 79 for rotation about an axis A as shown in Fig. 19. Motors, pistons, or other actuation mechanisms move pedals 76, 78 relative to base 79.

In one illustrative embodiment, movable foot support 72 tilts the feet of occupant 90 to the right, pads 71, 75 along a right side of vehicle seat 92 soften, and pads 71, 75 along a left side of vehicle seat 92 harden to inform occupant 90 of a right turn and reposition the body of occupant 90 to absorb the forces of the turn. Similarly, the engagement system 18 repositions the body of occupant toward left side 108 of cabin 101 to indicate a left turn. In another illustrative embodiment, center pads 73 soften and center pads 77 harden to inform occupant 90 of acceleration of vehicle 100. Similarly, center pads 77 soften and center pads 73 harden to inform occupant 90 of deceleration of vehicle 100.

An illustrative process 200 in accordance with the present invention for using motion-sickness mitigation system 10 is shown in Fig. 20. The process starts at step 201 and moves to step 202 where motion-sickness mitigation system 10 monitors for motion inputs 15 using control system 11. Monitoring is continued if no motion inputs 15 are detected as suggested at step 204. In response to a motion input 15 being detected, control system 11 identifies the type of change in movement 13 based on the detected motion input 15 as suggested at step 206. For example, rotating the steering wheel to the right leads to a change in movement 13 of a right turn. A signal indicating change in movement 13 is sent by control system 11 to sensory feedback system 12 as suggested at step 208. Sensory feedback system 12 generates an alert indicative of change in movement 13 to inform occupant 90 so that a likelihood of occupant 90 becoming motion-sick is reduced as suggested at step 209. Control system 11 continues to monitor for changes in movement 13.

In illustrative embodiments, motion-sickness mitigation system 10 is useful in reducing a likelihood that an occupant 90 of a vehicle 100 becomes motion sick due to changes in movement 13 of vehicle 100. Occupants can become distracted and unaware of their surroundings, such as by reading or watching content on an electronic device, which can lead to the onset of motion-sickness due to unknown changes in movement of the vehicle they are riding in. This is especially true with the rise of autonomous vehicles. Motion-sickness mitigation system 10 generates alerts to inform occupant 90 of changes in movement 13 to reduce the likelihood of occupant 90 becoming motion sick
The embodiments according to figures 1A to 11A do not form part of the invention but present examples useful to understand the invention.

Occupant supports 10A, 210A, 310A, 410A, 510A adapted for use in a vehicle 11A are shown in Figs. 1A, 8A, 9A, 10A, and 11A. Occupant supports 10A, 210A, 310A, 410A, 510A may mitigate, eliminate, or prevent motion sickness of an occupant supported by the occupant support 10A, 210A, 310A, 410A, 510A by reducing acceleration forces experienced by the occupant due to vehicle acceleration in the fore-and-aft and lateral axes.

Occupant supports 10A, 210A, 310A, 410A, 510A allow the occupant to move slightly laterally and fore and aft to more closely match the motion of the vehicle as suggested in Figs. 2A and 3A instead of remaining relatively static. As a result, the acceleration forces experienced by the occupant are reduced. This may also be referred to as G-force dampening. Motion sickness may result from a discrepancy between what our eyes perceive compared to what our body experiences (biological accelerometers: vestibular system, somatic, visceral).

Occupant support 10A includes a seat base 12A, a seat 14A, and motion-sickness mitigation means 16A as shown in Figs. 1A-3A and 6A. Seat base 12A is adapted to couple with a vehicle 11A for movement with vehicle 11A. Seat 14A is coupled with seat base 12A for movement with seat base 12A. Motion-sickness mitigation means 16A facilitates relative movement between seat base 12A and a support surface 30A of seat 14A in response to an acceleration of seat base 12A to reduce at least one of lateral acceleration and fore and aft acceleration of the occupant during acceleration of seat base 12A so that motion sickness experienced by the occupant is minimized as suggested in Figs. 2A and 3A.

Seat base 12A is configured to couple occupant support 10A with vehicle 11A as suggested in Fig. 1. In the illustrative embodiment, seat base 12A includes a plurality of mounting pads as shown in Fig. 1. In other embodiments, seat base 12A includes a rail system. Seat base 12A is coupled to a floor 13A of vehicle 11A in the illustrative embodiment. In other embodiments, seat base 12A is coupled to a frame arranged to extend around seat 14A as suggested in Fig. 10A. Seat base 12A moves with vehicle 11A such that acceleration of vehicle 11A is applied to seat base 12A.

Seat 14A includes a seat bottom 18A and a seat back 20A as shown in Figs. 1A-3A. Seat bottom 18A is adapted to support thighs of the occupant and seat back 20A is adapted to support a back and shoulders of the occupant. Seat bottom 18A includes a left lateral side 22A, a right lateral side 24A spaced apart from left lateral side 22A, a fore end 26A, and an aft end 28A spaced apart from fore end 26A. Seat back 20A is arranged to extend upwardly and away from aft end 28A of seat bottom 18A.

Seat bottom 18A and seat back 20A cooperate to define support surface 30A adapted to support the occupant of occupant support 10A thereon as shown in Fig. 1A. Support surface 30A is defined by a trim of seat 14A in the illustrative embodiment. Support surface 30A is configured to move relative to seat base 12A to mitigate motion sickness. Support surface 30A may move relative to seat base 12A due to movement of seat bottom 12A. Support surface 30A may move relative to seat base 12A in response to movement of support surface 30A relative to seat bottom 18A, for example, due to an adjustable seat cushion.

Motion-sickness mitigation means 16A couples seat bottom 18A with seat back 20A so that seat 14A moves with seat base 12A and vehicle 11A as suggested in Fig. 1A. Motion-sickness mitigation means 16A facilitates relative movement between support surface 30A of seat 14A and seat base 12A in response to an acceleration of seat base 12A to reduce at least one of lateral acceleration and fore-and-aft acceleration of the occupant during acceleration of seat base 12A so that motion sickness experienced by the occupant is mitigated, remedied, or prevented. In some embodiments, motion-sickness mitigation means 16A reduces vibrations of about 0.2 to about 0.4 Hertz. In some embodiments, motion-sickness mitigation means 16A reduces accelerations of about 0.1 to about 0.5 Hertz.

Motion-sickness mitigation means 16A includes a motion-sickness mitigation system 32A that includes a plurality of actuators 34A and a controller 36A as shown in Figs. 2A and 3A. The plurality of actuators 34A are coupled with seat bottom 18A and seat base 12A. Controller 36A is configured to control movement of each of the plurality of actuators 34A individually to cause the plurality of actuators 34A to move seat 14A relative to the seat base 12A. Actuators 34A may comprise air cylinders or air springs.

Acceleration of vehicle 11A is applied to the occupant through occupant support 10A as suggested in Figs. 4A and 5A. During a vehicle acceleration event, the momentum of the occupant resists the acceleration as suggested in Figs. 4A and 5A. This may cause the occupant to feel a pressure of their body acting against seat 14A. The motion-sickness mitigation means 16A of the present embodiment not forming part of the invention allows pendulum like movement of support surface 30A and the occupant relative to seat base 12A and floor 13A to reduce the magnitude of the acceleration felt by the occupant.

Motion-sickness mitigation means 16A of the present embodiment not forming part of the invention allows movement of support surface 30A and the occupant relative to seat base 12A and floor 13A to extend the time that force is applied to the occupant to reduce the magnitude of the acceleration felt by the occupant. The movement of support surface 30A may allow forces to be applied downwardly through occupant's thighs and into seat bottom 18A and through the occupant's feet into floor 13A as compared to the forces being applied laterally and being resisted by the abdomen and core of the occupant.

The plurality of actuators 34A are configured to translate and tilt seat bottom 18A relative to seat base 12A as suggested in Figs. 2A and 3A. Controller 36A is configured to cause the plurality of actuators 34A to raise left lateral side 22A and lower right lateral side 24A of seat bottom 18A in response to seat base 12A experiencing a right turn. For example, seat base 12A experiences a right turn when vehicle 11A is making a right turn.

Controller 36A is configured to cause the plurality of actuators 34A to raise right lateral side 24A and lower left lateral side 22A of seat bottom 18A in response to seat base 12A experiencing a left turn. Controller 36A is configured to cause the plurality of actuators 34A to translate seat bottom 18A aft relative to seat base 12A in response to seat base 12A experiencing a forward acceleration. Controller 36A is configured to cause the plurality of actuators 34A to translate seat bottom 18A forward relative to seat base 12A in response to seat base 12A experiencing a rearward acceleration. Controller 36A is configured to cause the plurality of actuators 34A to move seat bottom 18A to a defaults position relative to seat base 12A after acceleration of seat base 12A is removed. Controller 36A is configured to cause the plurality of actuators 34A to rotate seat bottom 18A so that the fore end rises and the aft end lowers in response to seat base 12A experiencing a forward acceleration. Controller 36A may be configured to cause the plurality of actuators 34A to rotate seat bottom 18A so that the fore end rises and the aft end lowers in response to seat base 12A experiencing a rearward acceleration.

In some embodiments not forming part of the invention, actuators 34A each include a worm gear and a motor located within a housing. The controller 36A is connected to the motor of each actuator 34A to actuate the worm gear according to the sensed acceleration by the system.

A not forming part of the invention of using occupant support 10A includes a number of steps. In a first step, seat base 12A and seat 14A are provided. Seat 14A defines support surface 30A adapted to support the occupant thereon. In a second step, support surface 30A of seat 14A is moved relative to seat base 12A from a first position to a second position in response to an acceleration of seat base 12A during acceleration of seat base 12A.

The second step may include translating support surface 30A relative to seat base 12A. The second step may include tilting support surface 30A relative to seat base 12A. The method may further include a third step of moving support surface 30A from the second position to the first position after the acceleration of seat base 12A is removed. Controller 36A is configured to make adjustments to means 16A manually by the occupant or automatically by a vehicle system as suggested in Fig. 7A.

Another embodiment not forming part of the invention of an occupant support 210A is shown in Fig. 8A. Occupant support 210A is substantially similar to occupant support 10A shown in Figs. 1A-7A and described herein. Accordingly, similar reference numbers in the 20A0 series indicate features that are common between occupant support 10A and occupant support 210A. The description of occupant support 10A is incorporated by reference to apply to occupant support 210A, except in instances when it conflicts with the specific description and the drawings of occupant support 210A.

Occupant support 210A includes a seat base 212A, seat 14A, and motion-sickness mitigation means 216A as shown in Fig. 8A. Motion-sickness mitigation means 216A includes motion-sickness mitigation system that includes a seat mount 242A coupled with seat bottom 18A for movement with seat bottom 18A. Seat mount 242A is rotatably coupled with seat base 212A to allow seat bottom 18A to rotate relative to seat base 212A. In the illustrative embodiment, seat mount 242A is passive and is free to move relative to seat base 212A. In other embodiments, seat mount 242A is actively controlled and controller 36A is configured to control movement of seat mount 242A relative to seat base 212A.

Another embodiment not forming part of the invention of an occupant support 310A is shown in Fig. 9A. Occupant support 310A is substantially similar to occupant support 10A shown in Figs. 1A-7A and described herein. Accordingly, similar reference numbers in the 300 series indicate features that are common between occupant support 10A and occupant support 310A. The description of occupant support 10A is incorporated by reference to apply to occupant support 310A, except in instances when it conflicts with the specific description and the drawings of occupant support 310A.

Occupant support 310A includes seat base 12A, seat 14A, and motion-sickness mitigation means 316A as shown in Fig. 9A. Motion-sickness mitigation means 316A includes a motion-sickness mitigation system that includes bias member 344A coupled with seat 14A and seat base 12A. Bias member 344A is configured to deform in response to acceleration of seat base 12A to allow seat 14A to move relative to seat base 12A during acceleration of seat base 12A. In the illustrative embodiment bias member 344A comprises a spring. In the illustrative embodiment, bias member 344A is passive and is free to move relative to seat base 12A. In other embodiments, bias member 344A is actively controlled and controller 36A is configured to control movement of bias member 344A relative to seat base 12A.

Another not forming part of the invention of an occupant support 410A is shown in Fig. 10A. Occupant support 410A is substantially similar to occupant support 10A shown in Figs. 1A-7A and described herein. Accordingly, similar reference numbers in the 400 series indicate features that are common between occupant support 10A and occupant support 410A. The description of occupant support 10A is incorporated by reference to apply to occupant support 410A, except in instances when it conflicts with the specific description and the drawings of occupant support 410A.

Occupant support 410A includes seat base 412A, seat 14A, and motion-sickness mitigation means 416A as shown in Fig. 10A. Seat base 412A is arranged above seat 14A. Motion-sickness mitigation means 410 includes motion-sickness mitigation system that includes strap 446A coupled with seat 14A and seat base 412A. Straps 446A are configured to suspend seat 14A from seat base 412A so that seat 14A is free to move relative to seat base 412A. In other embodiments, straps 446A are actively controlled and controller 36A is configured to control movement of straps 446A relative to seat base 412A.

Another not forming part of the invention of an occupant support 510A is shown in Fig. 11A. Occupant support 510A is substantially similar to occupant support 10A shown in Figs. 1A-7A and described herein. Accordingly, similar reference numbers in the 500 series indicate features that are common between occupant support 10A and occupant support 510A. The description of occupant support 10A is incorporated by reference to apply to occupant support 510A, except in instances when it conflicts with the specific description and the drawings of occupant support 510A.

Occupant support 510A includes seat base 12A, seat 14A, and motion-sickness mitigation means 516A as shown in Fig. 11A. Motion-sickness mitigation means 510 includes motion-sickness mitigation system that includes an adjustable seat cushion 548A coupled to seat bottom 14. Adjustable seat cushion 548A is configured to move support surface 30A of seat 14A relative to the seat base 12A. In one example, adjustable seat cushion 548A is configured to raise and lower a first lateral side of seat bottom 18A relative to a second lateral side of seat bottom 18A.

In illustrative embodiments not forming part of the invention , a motion-sickness mitigation system 32A may be used with a vehicle seat 14A to minimize motion sickness experienced by an occupant resting on the vehicle seat 14A. The motion-sickness mitigation system 32A may include one or more gravity dampers (or G dampers). Fig. 4A is a diagrammatic view showing a body of an occupant counterbalances within a vehicle moving in the opposite direction. Fig. 5A is a diagrammatic view showing inertial force experienced by an occupant that is accelerating in an occupant support 10A. Figs. 6A-10A show a series of diagrammatic views showing different aspects of G damper mechanisms that may be used alone or in combination with one another.

Motion sickness is a condition which may include a number of associated symptoms. The rise of autonomous vehicles may lead to an increase in the number of occupants that experience motion sickness. Many activities unrelated to driving may have a worsening effect on the frequency and severity of motion sickness symptoms. For example, motion sickness may occur when attempting to read or watch screen content while moving. It is desired to address motion sickness and minimize or eliminate the effects of its symptoms.

Various symptoms of motion sickness that may be alleviated by one or more embodiments of the present disclosure include cold sweating, increased salivation, pallor (skin color), drowsiness, headache, severe pain, nausea, vomiting, and sopite syndrome (which includes profound drowsiness and persistent fatigue which may result in hours or days of boredom, apathy, increased irritability, and personality changes). The symptoms mentioned above may have a response dependent upon the provocativeness of stimulation, relative susceptibility of the person, and prior experiences.

Results from motion sickness field testing show an abrupt duration of less than two seconds in changes of x- G forces and y-G forces. G force loading was less than 1 G in each axis with rapid combined x-G forces and y-G forces provoking motion sickness within two seconds. Each test subject experienced high motion sickness for about half of the testing time and each subject noted that they would not have continued reading as long as they did during the test. An S-curve including slight rolling hills through a dark tunnel provoked motion sickness in some cases. Test subjects experienced some level of residual motion sickness symptoms following testing.

The present disclosure provides systems to mitigate or eliminate motion sickness by reducing the effects of acceleration or deceleration and reducing or controlling vehicle vibration levels either within the vehicle suspension or within the seating system. A gravity damper (or G damper) system may provide partial car motion cancellation relative to the occupant, may absorb inertial forces to provide a constant state to the body of occupant, and may reduce or override uncomfortable vibrations that may result in motion sickness.

Fig. 6A depicts an embodiment of a G damper with pistons or air springs. Fig. 8A depicts an embodiment of a G damper with a swaying or rotating base. Fig. 11A depicts an embodiment of a G damper with an adjustable seat cushion, which may include a connected sensor and controller to automatically adjust seat cushion angles. Fig. 7A depicts an embodiment of a G damper with a tablet or other object associated with the seating position. For example, micro-adjustments may be made manually by the occupant or automatically by the system by using connected sensors and a controller. Fig. 9A depicts an embodiment of a G damper with a vehicle seat spring. Fig. 10A depicts an embodiment of a G damper with vehicle seat straps. Vehicle seat straps may be made from a variety of material, including but not limited to rope, rubber, wire, chains, or any other material capable of holding or securing objects to one another.

Anyone may be vulnerable to the effects of motion sickness. It may occur even for blind people that experience visual or other sensory-dependent adaptation to the moving environment. Although complex interactions may not be fully understood because there are significant individual variations, there may be vestibular inputs and visceral inputs due to motion, and there may be a relationship between the head and the corresponding torso movements of a person. The sensory conflict theory provides that motion sickness may be caused by a discrepancy between the expected and actual sensory feedback. The sensory conflict theory may be the most widely-accepted theory and differentiates between somatic (muscles and joints) and visceral (gut) receptors which may act as biological accelerometers.

Motion-sickness mitigation means are based on the Newton Laws of Motion. When a force is applied on a freely moving object in order to accelerate, decelerate, or change its direction, an equal inertial force acts on the object in an opposite direction of the applied force. An occupant can experience or feel that inertial force, which is an indicator of motion for their vestibular system.

The inertial force is proportional to the acceleration, so reducing the acceleration decreases the inertia force. Decoupling the occupant support from the rest of the vehicle, may allow it to freely move in the opposite direction of the vehicle acceleration, thus reducing the acceleration sustained by the occupant body and as a result the inertial force and the perception of motion. Following this principle, this disclosure may enable the occupant support to have a certain degree of free motion relatively to the vehicle frame, to enable it to move in the opposite direction of the vehicle acceleration.

This degree of free motion may be controlled via some dampening solution, to avoid an abrupt stop when reaching the limit of the free motion. The direction of free motion may also be controlled to maximize the occupant stability during the movement. Enabling the occupant body to describe the trajectory of a pendulum may create a centrifugal force (perpendicular to the inertial force), promoting stability.

## Claims

1. A motion-sickness mitigation system (10) comprising
a vehicle (100),
a control system (11) configured to monitor for motion input (15) including at least one of a concurrent, an anticipated, or an instructed change in movement of the vehicle (100), the control system (11) configured to produce a signal indicative of the change in movement in response to detecting the motion input (15), and
a sensory feedback system (12) configured to generate at least one of a tactile, visual, audial, and olfactory alert indicative of the change in movement to inform an occupant (90) of the vehicle (100) of the change in movement in response to receiving the signal from the control system (11) to reduce a likelihood of the occupant (90) becoming motion-sick.

2. The motion-sickness mitigation system (10) of claim 1, wherein the sensory feedback system (12) includes a lighting system (20) configured to illuminate a right-side (106) portion of a cabin (101) of the vehicle (100) in response to the signal received from the control system (11) being indicative of a right turn of the vehicle (100), wherein the lighting system (20) is configured to illuminate a left-side (108) portion of the cabin (101) in response to the signal received from the control system (11) being indicative of a left turn of the vehicle (100), wherein the lighting system (20) is configured to provide a moving point of illumination from a front (102) of the cabin (101) toward a rear (104) of the cabin (101) in response to the signal received from the control system (11) being indicative of an increase in speed of the vehicle (100), and wherein the lighting system (20) is configured to provide a moving point of illumination from the rear (104) of the cabin (101) toward the front (102) of the cabin (101) in response to the signal received from the control system (11) being indicative of a decrease in speed of the vehicle (100).

3. The motion-sickness mitigation system (10) of claim 1 or 2, wherein the sensory feedback system (12) includes a lighting system (20) configured to illuminate a cabin (101) of the vehicle (100) with a first color (23, 25, 27), wherein the lighting system (20) is configured to illuminate the cabin (101) with a second color (23, 25, 27) different than the first color (23, 25, 27) in response to the signal received from the control system (11) being indicative of a change in speed of the vehicle (100), wherein the second color (23, 25, 27) has a relatively longer wavelength in the electromagnetic spectrum than the first color when the change in speed is an increase in speed, and wherein the second color (23, 25, 27) has a relatively shorter wavelength than the first color (23, 25, 27) when the change in speed is decrease in speed.

4. The motion-sickness mitigation system (10) of claim 1, 2 or 3, wherein the sensory feedback system (12) includes an indicator system (30) configured to provide indicia (32, 34) to the signal received from the control system (11) and wherein the indicia (32, 34) is one of a right-turn arrow, a left-turn arrow, an upward extending arrow, and a downward extending arrow.

5. The motion-sickness mitigation system (10) of one of the preceding claims, wherein the sensory feedback system (12) includes a sound system (40) configured to provide sound along a right-side (106) portion of a cabin (101) of the vehicle (100) in response to receiving the signal indicative of a right turn of the vehicle (100), wherein the sound system (40) is configured to provide sound along a left-side (108) portion of the cabin (101) in response to the signal received from the control system (11) being indicative of a left turn of the vehicle (100), wherein the sound system (40) is configured to provide sound of increasing volume in response to the signal received from the control system (11) being indicative of an increase in speed of the vehicle (100), and wherein the sound system (40) is configured to provide sound of decreasing volume in response to the signal received from the control system (11) being indicative of a decrease in speed of the vehicle (100).

6. The motion-sickness mitigation system (10) of one of the preceding claims, wherein the sensory feedback system (12) includes a ventilation system (16) configured to provide air flow along a right side (106) of the occupant (90) in response to the signal received from the control system (11) being indicative of a right turn of the vehicle (100), wherein the ventilation system (16) is configured to provide air flow along a left side (108) of the occupant (90) in response to the signal received from the control system (11) being indicative of a left turn of the vehicle (100), wherein the ventilation system (16) is configured to provide air flow of increasing volume in response to the signal received from the control system (11) being indicative of an increase in speed of the vehicle (100), and wherein the ventilation system (16) is configured to provide air flow of decreasing volume in response to the signal received from the control system (11) being indicative of a decrease in speed of the vehicle (100).

7. The motion-sickness mitigation system (10) of one of the preceding claims, wherein the sensory feedback system (12) includes an engagement system (18) that comprises a first plurality of inflatable bladders (62) arranged along a right-side portion of a vehicle (100) seat supporting the occupant (90) and a second plurality of inflatable bladders (64) arranged along a left-side (108) portion of the vehicle (100) seat, wherein the first plurality of inflatable bladders (62) is configured to inflate in response to the signal received from the control system (11) being indicative of a right turn of the vehicle (100), wherein the second plurality of inflatable bladders (64) is configured to inflate in response to the signal received from the control system (11) being indicative of a left turn of the vehicle (100), wherein the first and second plurality of inflatable bladders (62, 64) are configured to inflate in a sequence moving in a direction from a front (102) of the cabin (101) toward a rear (104) of the cabin (101) in response to the signal received from the control system (11) being indicative of an increase in speed of the vehicle (100), and wherein the first and second plurality of inflatable bladders (62, 64) are configured to inflate in a sequence moving in a direction from the rear (104) of the cabin (101) toward the front (102) of the cabin (101) in response to the signal received from the control system (11) being indicative of a decrease in speed of the vehicle (100).

8. The motion-sickness mitigation system (10) of one of the preceding claims, wherein the sensory feedback system (12) includes an engagement system (18) that comprises a first plurality of vibration generators arranged along a right-side (106) portion of a vehicle (100) seat (92) supporting the occupant (90) and a second plurality of vibration generators arranged along a left-side portion of the vehicle (100) seat (92), wherein the first plurality of vibration generators is configured to vibrate in response to the signal received from the control system (11) being indicative of a right turn of the vehicle (100), wherein the second plurality of vibration generators is configured to vibrate in response to the signal received from the control system (11) being indicative of a left turn of the vehicle (100), wherein the first and second plurality of vibration generators are configured to vibrate in a sequence moving in a direction from a front (102) of the cabin (101) toward a rear (104) of the cabin (101) in response to the signal received from the control system (11) being indicative of an increase in speed of the vehicle (100), and wherein the first and second plurality of vibration generators are configured to vibrate in a sequence moving in a direction from the rear (104) of the cabin (101) toward the front of the cabin (101) in response to the signal received from the control system (11) being indicative of a decrease in speed of the vehicle (100).

9. The motion-sickness mitigation system (10) of one of the preceding claims, further comprising a vehicle (100) seat (92) configured to support the occupant (90) on a support surface thereof, wherein the vehicle (100) seat (92) includes a seat bottom (94) and a seat back (96), wherein an engagement system (18) of the sensory feedback system (12) comprises variable engagement pads (74) included in right-side and left-side bolsters (71, 75) of the seat bottom (94) and seat back (96) and in central portions (73, 77) of the seat bottom (94) and seat back (96), wherein the variable engagement pads (71, 73, 75, 77) are configured to alter the support surface on the right-side bolsters (71, 75) in response to the signal received from the control system (11) being indicative of a right turn of the vehicle (100), wherein the variable engagement pads (71, 73, 75, 77) are configured to alter the support surface on the left-side bolsters (71, 75) in response to the signal received from the control system (11) being indicative of a left turn of the vehicle (100), wherein the variable engagement pads (71, 73, 75, 77) are configured to alter the support surface on the central portion (73, 77) of the seat back (96) in response to the signal received from the control system (11) being indicative of an increase in speed of the vehicle (100), and wherein the variable engagement pads (71, 73, 75, 77) are configured to alter the support surface on the central portion (73, 77) at bottom (94) in response to the signal received from the control system (11) being indicative of a decrease in speed of the vehicle (100).

10. A method for operating a motion-sickness mitigation system (10) of one of the preceding claims, the method comprising
monitoring with a control system (11) for motion inputs (15) including at least one of a concurrent, an anticipated, and an instructed change in movement of the vehicle (100),
producing with the control system (11) a signal indicative of the change in movement in response to detecting a motion input (15),
receiving with a sensory feedback system (12) the signal produced by the control system (11), and
generating with the sensory feedback system (12) an alert indicative of the change in movement to inform the occupant (90) of the change in movement in response to receiving the signal from the control system (11) to reduce a likelihood of the occupant (90) becoming motion-sick.

11. The method of claim 10, wherein generating the alert with the sensory feedback system (12) includes illuminating a right-side (106) portion of a cabin (101) of the vehicle (100) in response to the sensory feedback system (12) receiving a signal indicating a right turn of the vehicle (100), illuminating a left-side (108) portion of the cabin (101) in response to the sensory feedback system (12) receiving a signal indicating a left turn of the vehicle (100), providing a moving point of illumination from a front of the cabin (101) toward a rear (104) of the cabin (101) in response to the sensory feedback system (12) receiving a signal indicating an increase in speed of the vehicle (100), and providing a moving point of illumination from the rear (104) of the cabin (101) toward the front (102) of the cabin (101) in response to the sensory feedback system (12) receiving a signal indicating a decrease in speed of the vehicle (100).

12. The method of claim 10 or 11, wherein generating the alert with the sensory feedback system (12) includes
illuminating a cabin (101) of the vehicle (100) with a first color (23, 25, 27), and
illuminating the cabin (101) with a second color (23, 25, 27) different than the first color (23, 25, 27) in response to the sensory feedback system (12) receiving a signal indicating a change in speed of the vehicle (100),
wherein the second color (71, 73, 75) has a relatively longer wavelength in the electromagnetic spectrum than the first color (71, 73, 75) when the change in speed is an increase in speed, and wherein the second color (71, 73, 75) has a relatively shorter wavelength than the first color (71, 73, 75) when the change in speed is decrease in speed.

13. The method of claim 10, 11 or 12, wherein generating the alert with the sensory feedback system (12) includes providing indicia (32, 34) to the occupant (90) corresponding to the change in movement of the vehicle (100) in response to the signal received from the control system (11), and wherein the indicia (32, 34) is one of a right-turn arrow, a left-turn arrow, a upward extending arrow, and a downward extending arrow.

14. The method of one of the claims 10-13, wherein generating the alert with the sensory feedback system (12) includes providing sound along a right-side portion of a cabin (101) of the vehicle (100) in response to the sensory feedback system (12) receiving a signal indicating a right turn of the vehicle (100), providing sound along a left-side (108) portion of the cabin (101) in response to the sensory feedback system (12) receiving a signal indicating a left turn of the vehicle (100), providing sound of increasing volume in response to the sensory feedback system (12) receiving a signal indicating an increase in speed of the vehicle (100), and providing sound of decreasing volume in response to the sensory feedback system (12) receiving a signal indicating a decrease in speed of the vehicle (100).

15. The method of one of the claims 10-14, wherein generating the alert with the sensory feedback system (12) includes providing air flow (56) along a right side (106) of the occupant (90) in response to the sensory feedback system (12) receiving a signal indicating a right turn of the vehicle (100), providing air flow (58) along a left side (108) of the occupant (90) in response to the sensory feedback system (12) receiving a signal indicating a left turn of the vehicle (100), providing air flow (56, 58) of increasing volume in response to the sensory feedback system (12) receiving a signal indicating an increase in speed of the vehicle (100), and providing air flow (56, 58) of decreasing volume in response to the sensory feedback system (12) receiving a signal indicating a decrease in speed of the vehicle (100).

## Patentansprüche

1. System zur Abschwächung von Bewegungskrankheit (10), aufweisend:
ein Fahrzeug (100),
ein Steuersystem (11), das zum Überwachen auf eine Bewegungseingabe (15) eingerichtet ist, die eine gleichzeitige und/oder eine erwartete und/oder eine befohlenen Änderung der Bewegung des Fahrzeugs (100) beinhaltet, wobei das Steuersystem (11) zum Erzeugen eines Signals, das für die Änderung der Bewegung bezeichnend ist, als Reaktion auf Erkennen der Bewegungseingabe (15) eingerichtet ist, und
ein sensorisches Rückkopplungssystem (12), das zum Erzeugen einer taktilen und/oder einer visuellen und/oder einer auditiven und/oder einer olfaktorischen Warnmeldung eingerichtet ist, die für die Bewegungsänderung bezeichnend ist, um einen Insassen (90) des Fahrzeugs (100) als Reaktion auf den Empfang des Signals von dem Steuersystem (11) her auf die Bewegungsänderung aufmerksam zu machen, um die Wahrscheinlichkeit, dass der Insasse (90) bewegungskrank wird, zu verringern.

2. System zur Abschwächung von Bewegungskrankheit (10) nach Anspruch 1, wobei das sensorische Rückkopplungssystem (12) ein Beleuchtungssystem (20) aufweist, das zum Beleuchten eines rechtsseitigen (106) Teils eines Innenraums (101) des Fahrzeugs (100) als Reaktion darauf, dass das von dem Steuersystem (11) her empfangene Signal für ein Rechtsabbiegen des Fahrzeugs (100) bezeichnend ist, eingerichtet ist, wobei das Beleuchtungssystem (20) zum Beleuchten eines linksseitigen (108) Teils des Innenraums (101) als Reaktion darauf, dass das von dem Steuersystem (11) her empfangene Signal für ein Linksabbiegen des Fahrzeugs (100) bezeichnend ist, eingerichtet ist, wobei das Beleuchtungssystem (20) zum Bereitstellen eines sich bewegenden Beleuchtungspunkts von einem vorderen Bereich (102) des Innenraums (101) zu einem hinteren Bereich (104) des Innenraums (101) hin als Reaktion darauf, dass das von dem Steuersystem (11) her empfangene Signal für eine Erhöhung der Geschwindigkeit des Fahrzeugs (100) bezeichnend ist, eingerichtet ist und wobei das Beleuchtungssystem (20) zum Bereitstellen eines sich bewegenden Beleuchtungspunkts von dem hinteren Bereich (104) des Innenraums (101) zu dem vorderen Bereich (102) des Innenraums (101) hin als Reaktion darauf, dass das von dem Steuersystem (11) her empfangene Signal für eine Verringerung der Geschwindigkeit des Fahrzeugs (100) bezeichnend ist, eingerichtet ist.

3. System zur Abschwächung von Bewegungskrankheit (10) nach Anspruch 1 oder 2, wobei das sensorische Rückkopplungssystem (12) ein Beleuchtungssystem (20) aufweist, das zum Beleuchten eines Innenraums (101) des Fahrzeugs (100) mit einer ersten Farbe (23, 25, 27) eingerichtet ist, wobei das Beleuchtungssystem (20) zum Beleuchten des Innenraums (101) mit einer zweiten Farbe (23, 25, 27), die von der ersten Farbe (23, 25, 27) verschieden ist, als Reaktion darauf, dass das von dem Steuersystem (11) her empfangene Signal für eine Änderung der Geschwindigkeit des Fahrzeugs (100) bezeichnend ist, eingerichtet ist, wobei die zweite Farbe (23, 25, 27) eine relativ längere Wellenlänge im elektromagnetischen Spektrum besitzt als die erste Farbe, wenn die Änderung der Geschwindigkeit eine Erhöhung der Geschwindigkeit ist, und wobei die zweite Farbe (23, 25, 27) eine relativ kürzere Wellenlänge als die erste Farbe (23, 25, 27) besitzt, wenn die Änderung eine Verringerung der Geschwindigkeit ist.

4. System zur Abschwächung von Bewegungskrankheit (10) nach Anspruch 1, 2 oder 3, wobei das sensorische Rückkopplungssystem (12) ein Anzeigersystem (30) aufweist, das eingerichtet ist, ein Zeichen (32, 34) zu dem von dem Steuersystem (11) her empfangenen Signal bereitzustellen, und wobei das Zeichen (32, 34) eines von einem Rechtsabbiegungspfeil, einem Linksabbiegungspfeil, einem sich aufwärts erstreckenden Pfeil und einem sich abwärts erstreckenden Pfeil ist.

5. System zur Abschwächung von Bewegungskrankheit (10) nach einem der vorhergehenden Ansprüche, wobei das sensorische Rückkopplungssystem (12) eine Tonanlage (40) aufweist, die eingerichtet ist, als Reaktion auf das Empfangen des Signals, das für ein Rechtsabbiegen des Fahrzeugs (100) bezeichnend ist, an einem rechtsseitigen (106) Teil eines Innenraums (101) des Fahrzeugs (100) entlang Ton bereitzustellen, wobei die Tonanlage (40) eingerichtet ist, als Reaktion darauf, dass das von dem Steuersystem (11) her empfangene Signal für ein Linksabbiegen des Fahrzeugs (100) bezeichnend ist, an einem linksseitigen (108) Teil des Innenraums (101) entlang Ton bereitzustellen, wobei die Tonanlage (40) eingerichtet ist, als Reaktion darauf, dass das von dem Steuersystem (11) her empfangene Signal für eine Erhöhung der Geschwindigkeit des Fahrzeugs (100) bezeichnend ist, Ton mit zunehmender Lautstärke bereitzustellen, und wobei die Tonanlage (40) eingerichtet ist, als Reaktion darauf, dass das von dem Steuersystem (11) her empfangene Signal für eine Verringerung der Geschwindigkeit des Fahrzeugs (100) bezeichnend ist, Ton mit abnehmender Lautstärke bereitzustellen.

6. System zur Abschwächung von Bewegungskrankheit (10) nach einem der vorhergehenden Ansprüche, wobei das sensorische Rückkopplungssystem (12) ein Lüftungssystem (16) aufweist, das eingerichtet ist, als Reaktion darauf, dass das von dem Steuersystem (11) her empfangene Signal für ein Rechtsabbiegen des Fahrzeugs (100) bezeichnend ist, an einer rechten Seite (106) des Insassen (90) entlang einen Luftstrom bereitzustellen, wobei das Lüftungssystem (16) eingerichtet ist, als Reaktion darauf, dass das von dem Steuersystem (11) her empfangene Signal für ein Linksabbiegen des Fahrzeugs (100) bezeichnend ist, an einer linken Seite (108) des Insassen (90) entlang einen Luftstrom bereitzustellen, wobei das Lüftungssystem (16) eingerichtet ist, als Reaktion darauf, dass das von dem Steuersystem (11) her empfangene Signal für eine Erhöhung der Geschwindigkeit des Fahrzeugs (100) bezeichnend ist, einen Luftstrom mit zunehmendem Volumen bereitzustellen, und wobei das Lüftungssystem (16) eingerichtet ist, als Reaktion darauf, dass das von dem Steuersystem (11) her empfangene Signal für eine Verringerung der Geschwindigkeit des Fahrzeugs (100) bezeichnend ist, einen Luftstrom mit abnehmendem Volumen bereitzustellen.

7. System zur Abschwächung von Bewegungskrankheit (10) nach einem der vorhergehenden Ansprüche, wobei das sensorische Rückkopplungssystem (12) ein Eingriffssystem (18) aufweist, das eine erste Vielzahl aufblasbarer Bälge (62), die an einem rechtsseitigen Teil eines den Insassen (90) tragenden Fahrzeug- (100) -sitzes entlang angeordnet sind, und eine zweite Vielzahl aufblasbarer Bälge (64), die an einem linksseitigen (108) Teil des Fahrzeug- (100) -sitzes entlang angeordnet sind, aufweist, wobei die erste Vielzahl aufblasbarer Bälge (62) eingerichtet ist, sich als Reaktion darauf, dass das von dem Steuersystem (11) her empfangenes Signal für ein Rechtsabbiegen des Fahrzeugs (100) bezeichnend ist, aufzublasen, wobei die zweite Vielzahl aufblasbarer Bälge (64) eingerichtet ist, sich als Reaktion darauf, dass das von dem Steuersystem (11) her empfangene Signal für ein Linksabbiegen des Fahrzeugs (100) bezeichnend ist, aufzublasen, wobei die erste und die zweite Vielzahl aufblasbarer Bälge (62, 64) eingerichtet sind, sich als Reaktion darauf, dass das von dem Steuersystem (11) her empfangene Signal für eine Erhöhung der Geschwindigkeit des Fahrzeugs (100) bezeichnend ist, in einer Reihenfolge aufzublasen, die sich in einer Richtung von einem vorderen Bereich (102) des Innenraums (101) zu einem hinteren Bereich (104) des Innenraums (101) hin bewegt, und wobei die erste und die zweite Vielzahl aufblasbarer Bälge (62, 64) eingerichtet sind, sich als Reaktion darauf, dass das von dem Steuersystem (11) her empfangene Signal für eine Verringerung der Geschwindigkeit des Fahrzeugs (100) bezeichnend ist, in einer Reihenfolge aufzublasen, die sich in einer Richtung von dem hinteren Bereich (104) des Innenraums (101) zu dem vorderen Bereich (102) des Innenraums (101) hin bewegen.

8. System zur Abschwächung von Bewegungskrankheit (10) nach einem der vorhergehenden Ansprüche, wobei das sensorische Rückkopplungssystem (12) ein Eingriffssystem (18) aufweist, das eine erste Vielzahl von Schwingungsgeneratoren, die an einem rechtsseitigen (106) Teil eines den Insassen (90) tragenden Fahrzeug- (100) -sitzes (92) entlang angeordnet sind, und eine zweite Vielzahl von Schwingungsgeneratoren, die an einem linksseitigen Teil des Fahrzeug- (100) -sitzes (92) entlang angeordnet sind, aufweist, wobei die erste Vielzahl von Schwingungsgeneratoren eingerichtet ist, als Reaktion darauf, dass das von dem Steuersystem (11) her empfangene Signal für ein Rechtsabbiegen des Fahrzeugs (100) bezeichnend ist, zu vibrieren, wobei die zweite Vielzahl von Schwingungsgeneratoren eingerichtet ist, als Reaktion darauf, dass das von dem Steuersystem (11) her empfangene Signal für ein Linksabbiegen des Fahrzeugs (100) bezeichnend ist, zu vibrieren, wobei die erste und die zweite Vielzahl von Schwingungsgeneratoren eingerichtet sind, als Reaktion darauf, dass das von dem Steuersystem (11) her empfangene Signal für eine Erhöhung der Geschwindigkeit des Fahrzeugs (100) bezeichnend ist, in einer Reihenfolge zu vibrieren, die sich in einer Richtung von einem vorderen Bereich (102) des Innenraums (101) zu einem hinteren Bereich (104) des Innenraums (101) hin bewegt, und wobei die erste und die zweite Vielzahl von Vibrationsgeneratoren eingerichtet sind, als Reaktion darauf, dass das von dem Steuersystem (11) her empfangene Signal für eine Verringerung der Geschwindigkeit des Fahrzeugs (100) bezeichnend ist, in einer Reihenfolge zu vibrieren, die sich in einer Richtung von dem hinteren Bereich (104) des Innenraums (101) zu dem vorderen Bereich des Innenraums (101) hin bewegt.

9. System zur Abschwächung von Bewegungskrankheit (10) nach einem der vorhergehenden Ansprüche, das ferner einen Fahrzeug- (100) -sitz (92) aufweist, der eingerichtet ist, den Insassen (90) auf einer Stützfläche des Sitzes zu tragen, wobei der Fahrzeug- (100) -sitz (92) ein Sitzunterteil (94) und eine Sitzrückenlehne (96) aufweist, wobei ein Eingriffssystem (18) des sensorischen Rückkopplungssystem (12) variable Eingriffspolster (74) aufweist, die in rechtsseitigen und linksseitigen Seitenwangen (71, 75) des Sitzunterteils (94) und der Sitzrückenlehne (96) und in mittleren Teilen (73, 77) des Sitzunterteils (94) und der Sitzrückenlehne (96) enthalten sind, wobei die variablen Eingriffspolster (71, 73, 75, 77) eingerichtet sind, die Stützfläche an den rechtsseitigen Seitenwangen (71, 75) als Reaktion darauf, dass das von dem Steuersystem (11) her empfangene Signal für eine Rechtsabbiegung des Fahrzeugs (100) bezeichnend ist, zu verändern, wobei die variablen Eingriffspolster (71, 73, 75, 77) eingerichtet sind, die Stützfläche an den linksseitigen Seitenwangen (71, 75) als Reaktion darauf, dass das von dem Steuersystem (11) her empfangene Signal für eine Linksabbiegung des Fahrzeugs (100) bezeichnend ist, zu verändern, wobei die variablen Eingriffspolster (71, 73, 75, 77) eingerichtet sind, die Stützfläche an dem mittleren Teil (73, 77) der Sitzrückenlehne (96) als Reaktion darauf, dass das von dem Steuersystem (11) her empfangene Signal für eine Erhöhung der Geschwindigkeit des Fahrzeugs (100) bezeichnend ist, zu verändern, und wobei die variablen Eingriffspolster (71, 73, 75, 77) eingerichtet sind, die Stützfläche an dem mittleren Teil (73, 77) am Unterteil (94) als Reaktion darauf, dass das von dem Steuersystem (11) her empfangene Signal für eine Verringerung der Geschwindigkeit des Fahrzeugs (100) bezeichnend ist, zu verändern.

10. Verfahren zum Betreiben eines Systems zur Abschwächung von Bewegungskrankheit (10) nach einem der vorhergehenden Ansprüche, wobei das Verfahren umfasst:
Überwachen, mit einem Steuersystem (11), auf Bewegungseingaben (15), die eine gleichzeitige und/oder eine erwartete und/oder eine befohlene Änderung der Bewegung des Fahrzeugs (100) beinhaltet,
Erzeugen, mit dem Steuersystem (11), eines Signals, das für die Änderung der Bewegung bezeichnend ist, als Reaktion auf Erkennen einer Bewegungseingabe (15);
Empfangen des von dem Steuersystem (11) erzeugten Signals mit einem sensorischen Rückkopplungssystem (12),
Erzeugen, mit dem sensorischen Rückkopplungssystem (12), einer Warnmeldung, die für die Bewegungsänderung bezeichnend ist, um den Insassen (90) als Reaktion auf den Empfang des Signals von dem Steuersystem (11) her aufmerksam zu machen, um die Wahrscheinlichkeit, dass der Insasse (90) bewegungskrank wird, zu verringern.

11. Verfahren nach Anspruch 10, wobei das Erzeugen der Warnmeldung mit dem sensorischen Rückkopplungssystem (12) umfasst: Beleuchten eines rechtsseitigen (106) Teils eines Innenraums (101) des Fahrzeugs (100) als Reaktion darauf, dass das sensorische Rückkopplungssystem (12) ein Signal empfängt, das ein Rechtsabbiegen des Fahrzeugs (100) anzeigt, Beleuchten eines linksseitigen (108) Teils des Innenraums (101) als Reaktion darauf, dass das sensorische Rückkopplungssystem (12) ein Signal empfängt, das ein Linksabbiegen des Fahrzeugs (100) anzeigt, Bereitstellen eines sich bewegenden Beleuchtungspunkts von einem vorderen Bereich des Innenraums (101) zu einem hinteren Bereich (104) des Innenraums (101) hin als Reaktion darauf, dass das sensorische Rückkopplungssystem (12) ein Signal empfängt, das eine Erhöhung der Geschwindigkeit des Fahrzeugs (100) anzeigt, und Bereitstellen eines sich bewegenden Beleuchtungspunkts von dem hinteren Bereich (104) des Innenraums (101) zu dem vorderen Bereich (102) des Innenraums (101) als Reaktion darauf, dass das sensorische Rückkopplungssystem (12) ein Signal empfängt, das eine Verringerung der Geschwindigkeit des Fahrzeugs (100) anzeigt.

12. Verfahren nach Anspruch 10 oder 11, wobei das Erzeugen der Warnmeldung mit dem sensorischen Rückkopplungssystem (12) umfasst:
Beleuchten eines Innenraums (101) des Fahrzeugs (100) mit einer ersten Farbe (23, 25, 27) und
Beleuchten des Innenraums (101) mit einer zweiten Farbe (23, 25, 27), die von der ersten Farbe (23, 25, 27) verschieden ist, als Reaktion darauf, dass das sensorische Rückkopplungssystem (12) ein Signal empfängt, das eine Änderung der Geschwindigkeit des Fahrzeugs (100) anzeigt,
wobei die zweite Farbe (71, 73, 75) eine relativ längere Wellenlänge im elektromagnetischen Spektrum hat als die erste Farbe (71, 73, 75), wenn die Änderung der Geschwindigkeit eine Erhöhung der Geschwindigkeit ist, und wobei die zweite Farbe (71, 73, 75) eine relativ kürzere Wellenlänge als die erste Farbe (71, 73, 75) hat, wenn die Änderung eine Verringerung der Geschwindigkeit ist.

13. Verfahren nach Anspruch 10, 11 oder 12, wobei das Erzeugen der Warnmeldung mit dem sensorischen Rückkopplungssystem (12) Bereitstellen eines Zeichens (32, 34) an den Insassen (90) umfasst, das der Änderung der Bewegung des Fahrzeugs (100) als Reaktion auf das von dem Steuersystem (11) empfangene Signal entspricht, und wobei das Zeichen (32, 34) eines von einem Rechtsabbiegungspfeil, einem Linksabbiegungspfeil, einem sich aufwärts erstreckenden Pfeil und einem sich abwärts erstreckenden Pfeil ist.

14. Verfahren nach einem der Ansprüche 10 bis 13, wobei das Erzeugen der Warnmeldung mit dem sensorischen Rückkopplungssystem (12) umfasst: Bereitstellen von Ton an einem rechtsseitigen Teil eines Innenraums (101) des Fahrzeugs (100) entlang als Reaktion darauf, dass das sensorische Rückkopplungssystem (12) ein Signal empfängt, das ein Rechtsabbiegen des Fahrzeugs (100) anzeigt, Bereitstellen von Ton an einem linksseitigen (108) Teil des Innenraums (101) entlang als Reaktion darauf, dass das sensorische Rückkopplungssystem (12) ein Signal empfängt, das ein Linksabbiegen des Fahrzeugs (100) anzeigt, Bereitstellen von Ton mit zunehmender Lautstärke als Reaktion darauf, dass das sensorische Rückkopplungssystem (12) ein Signal empfängt, das eine Verringerung der Geschwindigkeit des Fahrzeugs (100) anzeigt, und Bereitstellen von Ton mit abnehmender Lautstärke als Reaktion darauf, dass das sensorische Rückkopplungssystem (12) ein Signal empfängt, das eine Verringerung der Geschwindigkeit des Fahrzeugs (100) anzeigt.

15. Verfahren nach einem der Ansprüche 10 bis 14, wobei das Erzeugen der Warnmeldung mit dem sensorischen Rückkopplungssystem (12) umfasst: Bereitstellen eines Luftstroms (56) an einer rechten Seite (106) des Insassen (90) entlang als Reaktion darauf, dass das sensorische Rückkopplungssystem (12) ein Signal empfängt, das ein Rechtsabbiegen des Fahrzeugs (100) anzeigt, Bereitstellen eines Luftstroms (58) an einer linken Seite (108) des Insassen (90) entlang als Reaktion darauf, dass das sensorische Rückkopplungssystem (12) ein Signal empfängt, das ein Linksabbiegen des Fahrzeugs (100) anzeigt, Bereitstellen eines Luftstroms (56, 58) mit zunehmendem Volumen als Reaktion darauf, dass das sensorische Rückkopplungssystem (12) ein Signal empfängt, das eine Verringerung der Geschwindigkeit des Fahrzeugs (100) anzeigt, und Bereitstellen eines Luftstroms (56, 58) mit abnehmendem Volumen als Reaktion darauf, dass das sensorische Rückkopplungssystem (12) ein Signal empfängt, das eine Verringerung der Geschwindigkeit des Fahrzeugs (100) anzeigt.

## Revendications

1. Système (10) d'atténuation du mal des transports, le système comprenant
un véhicule (100),
un système de commande (11) conçu pour suivre une entrée de mouvement (15) comprenant une variation concomitante, et/ou anticipée, et/ou prescrite du déplacement du véhicule (100), le système de commande (11) étant conçu pour produire un signal indiquant la variation du déplacement en réponse à la détection de l'entrée de mouvement (15), et
un système de rétroaction sensorielle (12) conçu pour produire une alerte tactile, et/ou visuelle, et/ou auditive, et/ou olfactive indiquant la variation du déplacement, afin d'informer un occupant (90) du véhicule (100) de la variation du déplacement en réponse à la réception du signal à partir du système de commande (11), en vue de réduire la probabilité que l'occupant (90) subisse le mal des transports.

2. Le système (10) d'atténuation du mal des transports de la revendication 1, dans lequel le système de rétroaction sensorielle (12) comprend un système d'éclairage (20) conçu pour éclairer une partie côté droit (106) d'une cabine (101) du véhicule (100) en réponse au signal reçu à partir du système de commande (11) indiquant un virage à droite du véhicule (100), le système d'éclairage (20) étant conçu pour éclairer une partie côté gauche (108) de la cabine (101) en réponse au signal reçu à partir du système de commande (11) indiquant un virage à gauche du véhicule (100), le système d'éclairage (20) étant conçu pour produire un point d'éclairage se déplaçant d'une partie avant (102) de la cabine (101) vers une partie arrière (104) de la cabine (101) en réponse au signal reçu à partir du système de commande (11) indiquant une augmentation de la vitesse du véhicule (100), et le système d'éclairage (20) étant conçu pour produire un point d'éclairage se déplaçant de la partie arrière (104) de la cabine (101) vers la partie avant (102) de la cabine (101) en réponse au signal reçu à partir du système de commande (11) indiquant une diminution de la vitesse du véhicule (100).

3. Le système (10) d'atténuation du mal des transports des revendications 1 ou 2, dans lequel le système de rétroaction sensorielle (12) comprend un système d'éclairage (20) conçu pour éclairer une cabine (101) du véhicule (100) avec une première couleur (23, 25, 27), le système d'éclairage (20) étant conçu pour éclairer la cabine (101) avec une seconde couleur (23, 25, 27) différente de la première couleur (23, 25, 27) en réponse au signal reçu à partir du système de commande (11) indiquant une variation de la vitesse du véhicule (100), la seconde couleur (23, 25, 27) présentant une longueur d'onde dans le spectre électromagnétique relativement plus longue que la première couleur lorsque la variation de la vitesse est une augmentation de la vitesse, et la seconde couleur (23, 25, 27) présentant une longueur d'onde relativement plus courte que la première couleur (23, 25, 27) lorsque la variation de la vitesse est une diminution de la vitesse.

4. Le système (10) d'atténuation du mal des transports des revendications 1, 2 ou 3, dans lequel le système de rétroaction sensorielle (12) comprend un système indicateur (30) conçu pour apporter des indices (32, 34) au signal reçu à partir du système de commande (11), et les indices (32, 34) étant une flèche de virage à droite, une flèche de virage à gauche, une flèche s'étendant vers le haut ou une flèche s'étendant vers le bas.

5. Le système (10) d'atténuation du mal des transports de l'une quelconque des revendications précédentes, dans lequel le système de rétroaction sensorielle (12) comprend un système sonore (40) conçu pour produire un son le long d'une partie côté droit (106) d'une cabine (101) du véhicule (100) en réponse à la réception du signal indiquant un virage à droite du véhicule (100), le système sonore (40) étant conçu pour produire un son le long d'une partie côté gauche (108) de la cabine (101) en réponse au signal reçu à partir du système de commande (11) indiquant un virage à gauche du véhicule (100), le système sonore (40) étant conçu pour produire un son de volume croissant en réponse au signal reçu à partir du système de commande (11) indiquant une augmentation de la vitesse du véhicule (100), et le système sonore (40) étant conçu pour produire un son de volume décroissant en réponse au signal reçu à partir du système de commande (11) indiquant une diminution de la vitesse du véhicule (100).

6. Le système (10) d'atténuation du mal des transports de l'une quelconque des revendications précédentes, dans lequel le système de rétroaction sensorielle (12) comprend un système de ventilation (16) conçu pour produire un écoulement d'air le long d'un côté droit (106) de l'occupant (90) en réponse au signal reçu à partir du système de commande (11) indiquant un virage à droite du véhicule (100), le système de ventilation (16) étant conçu pour produire un écoulement d'air le long d'un côté gauche (108) de l'occupant (90) en réponse au signal reçu à partir du système de commande (11) indiquant un virage à gauche du véhicule (100), le système de ventilation (16) étant conçu pour produire un écoulement d'air de volume croissant en réponse au signal reçu à partir du système de commande (11) indiquant une augmentation de la vitesse du véhicule (100), et le système de ventilation (16) étant conçu pour produire un écoulement d'air de volume décroissant en réponse au signal reçu à partir du système de commande (11) indiquant une diminution de la vitesse du véhicule (100).

7. Le système (10) d'atténuation du mal des transports de l'une quelconque des revendications précédentes, dans lequel le système de rétroaction sensorielle (12) comprend un système de déclenchement (18) comportant une première pluralité de vessies gonflables (62) agencée le long d'une partie côté droit d'un siège du véhicule (100) portant l'occupant (90), et une seconde pluralité de vessies gonflables (64) agencée le long d'une partie côté gauche (108) du siège du véhicule (100), la première pluralité de vessies gonflables (62) étant conçue pour se gonfler en réponse au signal reçu à partir du système de commande (11) indiquant un virage à droite du véhicule (100), la seconde pluralité de vessies gonflables (64) étant conçue pour se gonfler en réponse au signal reçu à partir du système de commande (11) indiquant un virage à gauche du véhicule (100), les première et seconde pluralités de vessies gonflables (62, 64) étant conçues pour se gonfler selon une séquence se déplaçant dans une direction allant d'une partie avant (102) de la cabine (101) vers une partie arrière (104) de la cabine (101) en réponse au signal reçu à partir du système de commande (11) indiquant une augmentation de la vitesse du véhicule (100), et les première et seconde pluralités de vessies gonflables (62, 64) étant conçues pour se gonfler selon une séquence se déplaçant dans une direction allant de la partie arrière (104) de la cabine (101) vers la partie avant (102) de la cabine (101) en réponse au signal reçu à partir du système de commande (11) indiquant une diminution de la vitesse du véhicule (100).

8. Le système (10) d'atténuation du mal des transports de l'une quelconque des revendications précédentes, dans lequel le système de rétroaction sensorielle (12) comprend un système de déclenchement (18) comportant une première pluralité de générateurs de vibrations agencée le long d'une partie côté droit (106) d'un siège (92) du véhicule (100) portant l'occupant (90), et une seconde pluralité de générateurs de vibrations agencée le long d'une partie côté gauche du siège (92) du véhicule (100), la première pluralité de générateurs de vibrations étant conçue pour vibrer en réponse au signal reçu à partir du système de commande (11) indiquant un virage à droite du véhicule (100), la seconde pluralité de générateurs de vibrations étant conçue pour vibrer en réponse au signal reçu à partir du système de commande (11) indiquant un virage à gauche du véhicule (100), les première et seconde pluralités de générateurs de vibrations étant conçues pour vibrer selon une séquence se déplaçant dans une direction allant d'une partie avant (102) de la cabine (101) vers une partie arrière (104) de la cabine (101) en réponse au signal reçu à partir du système de commande (11) indiquant une augmentation de la vitesse du véhicule (100), et les première et seconde pluralités de générateurs de vibrations étant conçues pour vibrer selon une séquence se déplaçant dans une direction allant de la partie arrière (104) de la cabine (101) vers la partie avant de la cabine (101) en réponse au signal reçu à partir du système de commande (11) indiquant une diminution de la vitesse du véhicule (100).

9. Le système (10) d'atténuation du mal des transports de l'une quelconque des revendications précédentes, comprenant en outre un siège (92) du véhicule (100) conçu pour porter l'occupant (90) sur une surface de support, le siège (92) du véhicule (100) comprenant une partie inférieure (94) de siège et un dossier (96) de siège, un système de déclenchement (18) du système de rétroaction sensorielle (12) comprenant des coussinets de déclenchement variable (74) compris dans des traversins côté droit et côté gauche (71, 75) de la partie inférieure (94) du siège et du dossier (96) du siège, et dans des parties centrales (73, 77) de la partie inférieure (94) du siège et du dossier (96) du siège, les coussinets de déclenchement variable (71, 73, 75, 77) étant conçus pour modifier la surface de support sur les traversins côté droit (71, 75) en réponse au signal reçu à partir du système de commande (11) indiquant un virage à droite du véhicule (100), les coussinets de déclenchement variable (71, 73, 75, 77) étant conçus pour modifier la surface de support sur les traversins côté gauche (71, 75) en réponse au signal reçu à partir du système de commande (11) indiquant un virage à gauche du véhicule (100), les coussinets de déclenchement variable (71, 73, 75, 77) étant conçus pour modifier la surface de support sur la partie centrale (73, 77) du dossier (96) de siège en réponse au signal reçu à partir du système de commande (11) indiquant une augmentation de la vitesse du véhicule (100), et les coussinets de déclenchement variable (71, 73, 75, 77) étant conçus pour modifier la surface de support sur la partie centrale (73, 77) au niveau de la partie inférieure (94) en réponse au signal reçu à partir du système de commande (11) indiquant une diminution de la vitesse du véhicule (100).

10. Procédé d'exploitation d'un système (10) d'atténuation du mal de transports selon l'une quelconque des revendications précédentes, le procédé comprenant
le suivi, à l'aide d'un système de commande (11), d'entrées de mouvement (15) comprenant une variation concomitante, et/ou anticipée, et/ou prescrite du déplacement du véhicule (100),
la production, à l'aide du système de commande (11), d'un signal indiquant la variation du déplacement en réponse à la détection d'une entrée de mouvement (15),
la réception, à l'aide d'un système de rétroaction sensorielle (12), d'un signal produit par le système de commande (11), et
la production, à l'aide du système de rétroaction sensorielle (12), d'une alerte indiquant la variation du déplacement, afin d'informer l'occupant (90) de la variation du déplacement en réponse à la réception du signal à partir du système de commande (11), en vue de réduire la probabilité que l'occupant (90) subisse le mal des transports.

11. Le procédé de la revendication 10, dans lequel la production de l'alerte à l'aide du système de rétroaction sensorielle (12) comprend l'éclairage d'une partie côté droit (106) d'une cabine (101) du véhicule (100) en réponse à un signal reçu par le système de rétroaction sensorielle (12) indiquant un virage à droite du véhicule (100), l'éclairage d'une partie côté gauche (108) de la cabine (101) en réponse à un signal reçu par le système de rétroaction sensorielle (12) indiquant un virage à gauche du véhicule (100), la production d'un point d'éclairage se déplaçant d'une partie avant de la cabine (101) vers une partie arrière (104) de la cabine (101) en réponse à un signal reçu par le système de rétroaction sensorielle (12) indiquant une augmentation de la vitesse du véhicule (100), et la production d'un point d'éclairage se déplaçant de la partie arrière (104) de la cabine (101) vers la partie avant (102) de la cabine (101) en réponse à un signal reçu par le système de rétroaction sensorielle (12) indiquant une diminution de la vitesse du véhicule (100).

12. Le procédé des revendications 10 ou 11, dans lequel la production de l'alerte à l'aide du système de rétroaction sensorielle (12) comprend
l'éclairage d'une cabine (101) du véhicule (100) avec une première couleur (23, 25, 27), et
l'éclairage de la cabine (101) avec une seconde couleur (23, 25, 27) différente de la première couleur (23, 25, 27) en réponse à un signal reçu par le système de rétroaction sensorielle (12) indiquant une variation de la vitesse du véhicule (100),
la seconde couleur (71, 73, 75) présentant une longueur d'onde dans le spectre électromagnétique relativement plus longue que la première couleur (71, 73, 75) lorsque la variation de la vitesse est une augmentation de la vitesse, et la seconde couleur (71, 73, 75) présentant une longueur d'onde relativement plus courte que la première couleur (71, 73, 75) lorsque la variation de la vitesse est une diminution de la vitesse.

13. Le procédé des revendications 10, 11 ou 12, dans lequel la production de l'alerte à l'aide du système de rétroaction sensorielle (12) comprend l'apport d'indices (32, 34) à l'occupant (90) correspondant à la variation du déplacement du véhicule (100) en réponse au signal reçu à partir du système de commande (11), et les indices (32, 34) étant une flèche de virage à droite, une flèche de virage à gauche, une flèche s'étendant vers le haut ou une flèche s'étendant vers le bas.

14. Le procédé de l'une quelconque des revendications 10-13, dans lequel la production de l'alerte à l'aide du système de rétroaction sensorielle (12) comprend la production d'un son le long d'une partie côté droit d'une cabine (101) du véhicule (100) en réponse à un signal reçu par le système de rétroaction sensorielle (12) indiquant un virage à droite du véhicule (100), la production d'un son le long d'une partie côté gauche (108) de la cabine (101) en réponse à un signal reçu par le système de rétroaction sensorielle (12) indiquant un virage à gauche du véhicule (100), la production d'un son de volume croissant en réponse à un signal reçu par le système de rétroaction sensorielle (12) indiquant une augmentation de la vitesse du véhicule (100), et la production d'un son de volume décroissant en réponse à un signal reçu par le système de rétroaction sensorielle (12) indiquant une diminution de la vitesse du véhicule (100).

15. Le procédé de l'une quelconque des revendications 10-14, dans lequel la production de l'alerte à l'aide du système de rétroaction sensorielle (12) comprend la production d'un écoulement d'air (56) le long d'un côté droit (106) de l'occupant (90) en réponse à un signal reçu par le système de rétroaction sensorielle (12) indiquant un virage à droite du véhicule (100), la production d'un écoulement d'air (58) le long d'un côté gauche (108) de l'occupant (90) en réponse à un signal reçu par le système de rétroaction sensorielle (12) indiquant un virage à gauche du véhicule (100), la production d'un écoulement d'air (56, 58) de volume croissant en réponse à un signal reçu par le système de rétroaction sensorielle (12) indiquant une augmentation de la vitesse du véhicule (100), et la production d'un écoulement d'air (56, 58) de volume décroissant en réponse à un signal reçu par le système de rétroaction sensorielle (12) indiquant une diminution de la vitesse du véhicule (100).
